(19) Europäisches Patentamt / European Patent Office / Office européen des brevets

(11) **EP 2 817 406 B1**

(12) **EUROPÄISCHE PATENTSCHRIFT**

(45) Veröffentlichungstag und Bekanntmachung des Hinweises auf die Patenterteilung:
**11.09.2019 Patentblatt 2019/37**

(51) Int Cl.:
*C12M 1/08* ^(2006.01)      *C12M 1/04* ^(2006.01)

(21) Anmeldenummer: **13707318.5**

(22) Anmeldetag: **20.02.2013**

(86) Internationale Anmeldenummer:
**PCT/EP2013/053390**

(87) Internationale Veröffentlichungsnummer:
**WO 2013/124326 (29.08.2013 Gazette 2013/35)**

(54) **EINWEG ABSCHEIDER ZUR RÜCKHALTUNG UND RÜCKFÜHRUNG VON ZELLEN**

DISPOSABLE CUTTER FOR RETAINING AND RETURNING CELLS

SÉPARATEUR À USAGE UNIQUE DESTINÉ À LA RÉTENTION ET AU RETOUR DE CELLULES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.02.2012 EP 12001121**

(43) Veröffentlichungstag der Anmeldung:
**31.12.2014 Patentblatt 2015/01**

(73) Patentinhaber: **Bayer Aktiengesellschaft 51373 Leverkusen (DE)**

(72) Erfinder:
• **KAULING, Joerg**
 **51427 Bergisch Gladbach (DE)**
• **SELETZKY, Juri**
 **Berkeley, CA 94710-2428 (US)**
• **MAGNUS, Jorgen**
 **40225 Düsseldorf (DE)**
• **PASTOR, Andre**
 **42719 Solingen (DE)**
• **BROD, Helmut**
 **51061 Köln (DE)**

(74) Vertreter: **BIP Patents c/o Bayer Intellectual Property GmbH Alfred-Nobel-Straße 10 40789 Monheim am Rhein (DE)**

(56) Entgegenhaltungen:
EP-A1- 0 471 947          EP-A2- 0 599 651
WO-A1-00/05337            WO-A1-98/07828
WO-A1-99/58222            WO-A1-2009/139703
WO-A1-2011/142670         WO-A2-03/020919
WO-A2-2009/152990         DE-A1- 2 548 950
DE-A1- 4 401 576          DE-A1- 10 223 536
DE-A1-102010 015 236      DE-U- 7 215 337
US-A- 4 814 278           US-A- 5 350 527
US-A- 5 698 102           US-B1- 6 959 618

**Beschreibung**

Die Erfindung betrifft einen Einweg Abscheider zur Rückhaltung und Rückführung von Zellen in einem kontinuierlich oder absatzweise durchströmten Kunststoffbeutel, der vorzugsweise außerhalb eines Bioreaktors betrieben werden kann. Die Erfindung betrifft weiterhin ein Verfahren zur Rückhaltung und Rückführung von Zellen innerhalb oder außerhalb des erfindungsgemäßen Bioreaktors. Ferner betrifft die Erfindung ein Verfahren zur Herstellung des erfindungsgemäßen Abscheiders.

Die Züchtung tierischer und pflanzlicher Zellen hat große Bedeutung bei der Herstellung biologisch aktiver Substanzen und pharmazeutisch aktiver Erzeugnisse. Insbesondere die Züchtung von Zellen, welche häufig in einem Nährmedium in freier Suspension durchgeführt wird, ist anspruchsvoll, weil die Zellen im Gegensatz zu Mikroorganismen sehr empfindlich hinsichtlich mechanischer Scherbeanspruchung und unzureichender Versorgung mit Nährstoffen sind.

Meist werden tierische und pflanzliche Zelllinien absatzweise gezüchtet. Dies hat den Nachteil, dass eine optimale Versorgung der Zellen infolge der sich ständig verändernden Substrat-, Produkt- und Biomassekonzentrationen nur schwer zu erreichen ist. Am Ende der Fermentation reichern sich außerdem Nebenprodukte an, z.B. Bestandteile abgestorbener Zellen, die meist unter großem Aufwand bei der späteren Aufarbeitung entfernt werden müssen. Aus den genannten Gründen, insbesondere aber bei der Herstellung instabiler Produkte, die z.B. durch proteolytische Angriffe beschädigt werden können, werden daher kontinuierlich betriebene Bioreaktoren verwendet.

Mit kontinuierlichen Bioreaktoren lassen sich hohe Zelldichten und eine damit verbundene hohe Produktivität erreichen, wenn folgende Anforderungen erfüllt sind:

- eine ausreichende und scherarme Versorgung der Zellen mit Substraten, insbesondere dem gelöstem Sauerstoff,

- eine ausreichende Entsorgung des bei der Veratmung entstehenden Kohlendioxids,

- ein effektives, scherarmes, verstopfungssicheres Zellrückhaltesystem zum Aufbau hoher Zellkonzentrationen,

- die Langzeitstabilität (Sterilität, Hydrodynamik) des Bioreaktors und Rückhaltesystems.

Neben der kontinuierlichen Fahrweise kann ein Bioreaktor mit einem effizienten Zellrückhaltesystem z.B. auch zur Anzucht von Vorkulturen mit besonders hohen Zelldichten verwendet werden. Man verwendet dann das Zellrückhaltesystem auf diskontinuierliche Weise im Repeated---Batch----Modus, um Zellkulturüberstand nahezu frei von Biomasse abzuziehen. Danach kann der Vorkulturreaktor wieder mit frischem Nährmedium aufgefüllt werden, um die Kultur auf diese Weise zu höheren Zelldichten zu bringen als bei einfachem absatzweisen Betrieb.

Damit eine hohe Zelldichte (> 20 Millionen lebende Zellen pro Milliliter) in einem kontinuierlich betriebenen Bioreaktor erreicht werden kann, ist eine effiziente Rückhaltung der Zellen notwendig. Der erforderliche Rückhaltegrad hängt dabei von der Wachstumsrate der Zellen und der Perfusionsrate $q/V$ (Mediendurchsatz q pro Bioreaktorvolumen $V$) ab.

In der Vergangenheit wurden unterschiedliche Zellrückhaltesysteme für kontinuierlich betriebene Bioreaktoren vorgeschlagen, welche zumeist außerhalb des Bioreaktors angeordnet werden. Grund hierfür ist die leichte Zugänglichkeit des Zellrückhaltesystems zu Wartungs- und Reinigungszwecken.

Um Schädigungen der Zellen insbesondere aufgrund unzureichender Sauerstoffversorgung und Kohlendioxidentsorgung außerhalb des Bioreaktors sowie eine Wirkstoffdegradation durch enzymatische Angriffe so klein wie möglich zu halten, sind Zellrückhaltesysteme mit kleinen Arbeitsvolumina und damit verbundenen kurzen Verweilzeiten der Zellen wünschenswert.

Neben Membranfiltern, Apparaten, die nach dem Prinzip der Querstromfiltration mit feststehenden und beweglichen Membranen arbeiten, kommen im Stand der Technik spezielle Zentrifugen und Schwerkraftabscheider zum Einsatz.

Im Fall der Zellrückhaltung mit Hilfe von Membranfiltern werden Ablagerungen bzw. Verschmutzungen beobachtet, die einen zuverlässigen und wartungsfreien Langzeitbetrieb verhindern können. Die Ablagerungen können reduziert werden, wenn die Membranflächen hinreichend schnell überströmt werden. Dies kann in stationärem oder oszillierendem Betrieb erreicht werden. Ein Beispiel für ein oszillierend überströmtes Membransystem ist das Alternating Tangential Flow (ATF) System der Firma Refine Technologies Inc.). Die schnelle Überströmung der Membranflächen läuft jedoch der Grundvoraussetzung an eine scherarme Zellkultivierung zuwider.

Scherarme Zentrifugen zur Abscheidung von Zellen im Zentrifugalfeld arbeiten nur über wenige Wochen ohne Wartungsaufwand und machen einen Austausch der Zentrifugenelementen erforderlich. Hiermit vergrößert sich das Risiko von Kontaminationen.

Die bei der Züchtung von Zellen überwiegend verwendeten Schwerkraftabscheider sind Absetzbehälter und Schrägkanalabscheider. Verglichen mit einfachen Absetzbehältern haben die Schrägkanalabscheider in großen Maßstäben den Vorteil eines beträchtlich geringeren Volumens in Verhältnis zur Abscheidefläche. Eine Veröffentlichung (Henzler, H.-J., Chemie-Technik, 1, 1992, 3) beschreibt die Zellrückhaltung in Schrägkanalabscheidern, welche im Gegenstrom, Kreuzstrom und Gleichstrom betrieben werden können. Der durchströmte Kanalquerschnitt kann mit Platten oder Rohren versehen sein. Die Patentschriften US 5,817,505 und EP 0 699 101 B1 beanspruchen den Einsatz von Schrägkanalabscheidern zur Rückhaltung von Zellen in Gegenstromabscheidern. In WO2003020919 A2 werden unter anderem Gegen- und Kreuzstromabscheider, sowie Kombinationen mit verschiedenen Vorabscheidern (z.B. Hydrozyklonen) für die Rückhaltung von Zellen beschrieben.

Die Schrägkanalabscheider werden über einen externen Kreislauf an den Bioreaktor angeschlossen. Hierzu sind Schlauchleitungen und Pumpen erforderlich.

Um die Stoffwechselaktivität und das Anbacken von Zellen in einem Schwerkraftabscheider zu vermindern, wird das Herunterkühlen der Zellkulturbrühe auf ihrem Weg zum Schwerkraftabscheider vorgeschlagen. Eine reduzierte Stoffwechselaktivität bei niedriger Temperatur ist bei längerem Aufenthalt der Zellen außerhalb des Bioreaktors sicherlich von Vorteil.

DE1022353 offenbart ein Verfahren zur Trennung nicht-adhärenter Zellen von adhärenten Zellen durch Nutzung der selektiven Adhärenz und der Schwerkraft, wobei bei der Trennung das Auftreten von Scherkräften im wesentlichen vermieden wird.

WO2009152990(A2) beschreibt ein Zellrückhaltesystem zur Rückhaltung und Rückführung von Zellen in einem durchströmten Gefäß umfassend eine Vielzahl von nebeneinander angeordneten Kanälen, wobei die Kanäle einen stehenden Hohlzylinder bilden und gegenüber der Längsachse des Hohlzylinders um einen Winkel $\beta$ zwischen 10° und 60°, geneigt sind. Das durchströmte Gefäß kann ein Bioreaktor oder ein mit einem Bioreaktor verbundenes Gefäß zur Zellrückhaltung und - rückführung sein. Die Kanäle sind am unteren Ende geöffnet. Am oberen Ende führen sie in einen gemeinsamen Ringraum, der über mindestens eine Leitung verfügt, über die ein Erntestrom aus dem Gefäß befördert werden kann. In den Kanälen erfolgt die Separation von Zellen und Zellkulturlösung. Durch die kontinuierliche Entnahme des Erntestroms aus dem Bioreaktor werden Zellkulturlösung und Zellen in die Kanäle eingesaugt. Die Zellen sedimentieren innerhalb der schräg angeordneten Kanäle und rutschen wie in klassischen Schrägkanalabscheidern im Gegenstrom zum zufließenden Erntestrom wieder aus den Kanälen heraus und verbleiben damit im Gefäß. Die von den Zellen getrennte Zellkulturlösung wird durch die Kanäle in den Ringraum oberhalb der Kanäle und schließlich aus dem Gefäß befördert.

Bei der stark regulierten pharmazeutischen Produktion entfällt ein großer zeitlicher, technischer und personeller Aufwand auf die Bereitstellung gereinigter und sterilisierter Bioreaktoren und Bioreaktorelemente wie z. B. Zellrückhaltesysteme. Um Kreuzkontaminationen bei einem Produktwechsel in einer Multi-Purpose-Anlage oder zwischen zwei Produktchargen sicher zu vermeiden, wird außer der Reinigung eine sehr aufwendige Reinigungsvalidierung benötigt, welche bei einer Prozessadaption ggf. wiederholt werden muss. Zur Reinigung und Sterilisation konventioneller Batch, Fed-Batch oder Perfusionsfermenter aus Edelstahl kommt in der Regel die Clean-in-Place (CIP)-Technik in Kombination mit der Steam-in-Place (SIP)-Technik in sog. fest verrohrten Anlagen zum Einsatz. Um bei kontinuierlicher Prozessführung eine ausreichende Langzeitsterilität zu gewährleisten, wird auch die Autoklavier-Technik genutzt, die allerdings einen umständlichen Transport der Reaktoren oder Reaktorelemente zum Autoklaven erfordert und nur in vergleichsweise kleinen Reaktormaßstäben anwendbar ist. Die Gefahr der Kontamination ist besonders kritisch bei Verwendung von alternden Verschleißteilen, z.B. gedichteten Rührerwellen, unsachgemäßer Sterilisation oder Anlagentransport, der Inbetriebnahme bzw. dem Verbinden von Anschlussleitungen nach Autoklavieren und der regelmäßigen Probeentnahme.

Bei im Batch- oder Fed-Batch-Modus genutzten CIP/SIP-Anlagen kann der durch die Bereitstellungsprozeduren bedingte Nutzungsausfall eines Reaktors insbesondere bei häufigem Produktwechsel infolge der kurzen Nutzungsperioden in der Größenordnung der Reaktorverfügbarkeit liegen.

Um der Forderung an ein schnelles und flexibles Neubeschicken der Produktionsanlage unter Wahrung maximaler Sauberkeit und Sterilität gerecht zu werden, erfreuen sich auf dem Markt Konzepte für Einweg-Reaktoren eines ständig wachsenden Interesses.

Es stellt sich damit ausgehend vom Stand der Technik die Aufgabe, eine effiziente Methode zur Rückhaltung und Rückführung von tierischen, insbesondere humanen, und pflanzlichen Zellen in einem kontinuierlich oder absatzweise betriebenen Verfahren bereitzustellen, die der Empfindlichkeit der Zellen hinsichtlich mechanischer Scherbeanspruchung und der ausreichenden Versorgung der Zellen mit Nährstoffen Rechnung trägt, die bis in sehr große Maßstäbe skalierbar ist, die den wartungs-, reinigungs- und sterilitätstechnischen Anforderungen der pharmazeutischen Industrie gerecht wird, deren Verwendung die Komplexität und das Fehlerrisiko erniedrigt, und die bei minimalem Ressourceneinsatz eine ökonomisch und ökologisch optimale Nutzung (Herstellung und Entsorgung) als Einwegsysteme ermöglicht.

Die oben genannte Aufgabe wurde durch einen Einweg-Zellenabscheider zur Rückhaltung und Rückführung von Zellen aus einem Bioreaktorgemisch gelöst, umfassend einen durchströmbaren, sterilisierbaren nach dem Stand der Technik z.B. bevorzugt gamma-bestrahlbaren, autoklavierbaren oder chemisch sterilisierbaren Kunststoffbeutel oder eine entsprechend behandelbare Kunststoffflasche mit folgenden Einbauten:

- Im oberen Bereich des Kunststoffbeutels oder der Kunststoffflasche ein oder mehreren Durchführungen/Einbauten (80) zum Abziehen eines von den Zellen getrennten Erntestromes (70) (=Harvest) aus einem Erntestromsammelbereich 56

- im oberen Segment eines mittleren Bereiches des Kunststoffbeutels einen Abscheidebereich umfassend eine Abscheidefläche (500) oder ein die Abscheidefläche enthaltendes Lamellenpaket (1), das während des Betriebs mit einem Winkel (10 = β) von 0° bis 80° zur Horizontalen geneigt ist,

- im unteren Segment des mittleren Bereiches des Kunststoffbeutels oder der Kunststoffflasche eine oder mehrere Durchführungen oder Einbauten (84), optional mit horizontalen Verteiler (85), zur gleichmäßigen horizontalen Strömungsverteilung der Zellkulturlösung (=Feed) (74) über eine Einleitfläche (510),

- Im unteren Bereich des Kunststoffbeutels oder der Kunststoffflasche einen unten konisch verjüngten Feststoffsammelbereich (57) zum Sammeln der Zellen mit Hilfe der Schwerkraft. Üblicherweise weist der Feststoffsammelbereich (57) eine oder mehrere Durchführungen (89) oder Einbauten (88) zum Abziehen der Zellen auf.

Gegenstand der Erfindung ist daher ein Feststoffabscheider zur Rückhaltung von Feststoffen aus einem Reaktorgemisch, umfassend einen durchströmten sterilisierbarenKunststoffbeutel oder Kunststoffflasche und innerhalb des Kunststoffbeutels oder der Kunststoffflasche:

- Im oberen Bereich ein oder mehreren Durchführungen/Einbauten (80) zum Abziehen eines von den Feststoffen getrennten Erntestrom (70) aus einem Erntestromsammelbereich (56),

- im oberen Segment eines mittleren Bereiches einen Abscheidebereich (1, 501) mit einer Abscheidefläche, die während des Betriebs mit einen Winkel (10 = β)von 0° bis 80° zur Horizontale geneigt ist,

- im unteren Segment des mittleren Bereiches eine oder mehrere Durchführungen oder Einbauten (84), optional mit horizontalen Verteiler (85), zur gleichmäßigen horizontalen Strömungsverteilung des Reaktorgemisches (74),

- Im unteren Bereich ein nach unten, üblicherweise konisch oder pyramidal, verjüngten Feststoffsammelbereich (57) zum Sammeln der Feststoffe mit Hilfe der Schwerkraft.

Üblicherweise weist der Feststoffsammelbereich (57) eine oder mehrere Durchführung (89) oder Einbauten (88) zum Abziehen der Feststoffe auf. Hiermit können die Feststoffe bei Bedarf in den Reaktor rückgeführt werden.

Bevorzugt weist der nach unten verjüngten Feststoffsammelbereich einen Winkel 58, 59 von 10° bis 60° gegen die Vertikale auf, wobei die Winkel 58 und 59 einzeln auswählbar sind.

Die Form des Erntestromsammelbereichs 56 kann beliebig insb. flach oder nach oben verjüngt sein.

Üblicherweise wird der Kunststoffbeutel oder die Kunststoffflasche aus einem ein- oder mehrlagigen durchsichtigen Polymermaterial ausgeführt, dass einen Einblick in die Vorrichtung während des Betriebs ermöglicht.

Das Polymermaterial des Kunststoffbeutels gestattet bei üblichen geringen Filmdicken von s < 1 mm Apparate mit einem vergleichsweise kleinem Masseanteil. Es ist kostengünstig zu beschaffen und zu verarbeiten, was sich sehr gut für den Aufbau von Einwegsystemen eignet. Die Entsorgung der gebrauchten Abscheider und die Verwendung eines neuen Einwegabscheiders sind damit wirtschaftlicher als die Reinigung von gebrauchten Abscheidevorrichtungen, insbesondere da bei der Verwendung von Einweg-Abscheidern eine teure Reinigung mit Wasser für Injektionen (Water for Injections, WFI) und die zeitaufwändige Reinigungsvalidierung entfällt. Der erfindungsgemäße Abscheider ist vorzugsweise für die Verbindung mit dem Bioreaktorsystem durch Schlauchleitungen mit entsprechenden steril kuppelbaren Verbindungselemente und Filterelementen geeignet assembliert und steril verpackt.

Als Materialien für den Kunststoffbeutel eignen sich insbesondere die in der Patentschrift US 6,186,932 B1 in den Spalten 2 und 3 für die dort genannten Transportbeutel (*sachets*) verwendeten Materialien und Materialkombinationen. Auch die dort aufgeführten Wandstärken lassen sich auf die erfindungsgemäße Abscheidevorrichtung übertragen.

In einer bevorzugten Ausführungsform bestehen die Wände des Kunststoffbeutels aus einem dem Fachmann bekannten Folienverbundmaterial aus zwei oder mehreren Schichten (Laminat oder Co-Extrudat), um die Eigenschaften des Kunststoffbeutels hinsichtlich Entfaltungsverhalten, Dehnungsverhalten, Gasdiffusion, Stabilität, Prozessverträglichkeit (minimale Adsorption von Produkten und Zellen) und Schweißbarkeit zu verbessern.

Der erfindungsgemäße Feststoffabscheider mit Kunststoffbeutel aus Polymerfolien kann beispielsweise nach dem in US 6,186,932 B1 beschriebenen Verfahren hergestellt werden, wobei die Schweißnähte angepasst werden müssen. Ausführungsbeispiele für die Herstellung von bevorzugten Ausführungsformen der erfindungsgemäßen Abscheidevorrichtung sind weiter unten beschrieben.

Durchführungen werden üblicherweise aus demselben Material hergestellt, aus dem auch die produktberührte Folie besteht, um mit dieser eine steril- und festigkeitstechnisch einwandfreie Verschweißung zu ermöglichen. Bevorzugtes produktberührtes Filmmaterial ist Polyethylen verschiedener dem Fachmann bekannter Vernetzungsgrade. Als Außenmantelfolien kommen je nach Anwendung und Prozessanforderung verschiedene dem Fachmann bekannte Materialien mit einem gegenüber der Innenfolie vergrößerten Schmelzpunkt für den Einsatz von thermischen Schweißverfahren und/oder besseren Festigkeits- und/oder Diffusionseigenschaften zum Einsatz.

Üblicherweise sind die Einbauten 80, 88 und 84 eingeschweißte Durchführungen, an die Leitungen, vorzugsweise Schläuche zur Verbindung mit dem Bioreaktor oder anderen externen Anlagen, angeschlossen werden können. Alternativ können die Durchführungen für das Hindurchtreten der Anschlussleitungen in eine oder mehrere Anschlussplatte(n), Deckel oder Stopfen eingebracht werden.

In einer besonderen Ausführungsform des erfindungsgemäßen Abscheiders befindet sich im oberen Bereich an der Wand des Kunststoffbeutels (Tetraeder) oder an einer Ecke (Würfel) eine oder mehrere Anschlussplatten 90, vorzugsweise eine, die auch ein Deckel sein kann, die die Durchführungen für das Hindurchtreten der Anschlussleitungen enthält und im Bereich des Anschlusses eine Halterung des Abscheiders ermöglicht. Sie wird üblicherweise bei der Montage mit dem Gehäuse des Abscheiders verbunden.

Alternativ kann am Ende des verjüngten Feststoffsammelbereichs 57 an einem Hals ein Deckel oder ein Stopfen 220 eingebracht werden. Deckel oder Stopfen umfassen in dieser Ausführungsform die Durchführungen für das Hindurchtreten der Anschlussleitungen.

Das Abziehen der Feststoffe aus dem Feststoffsammelbereich 57 erfolgt üblicherweise über eine oder mehrere Durchführungen bzw. Einbauten 88 in der Nähe der unteren Spitze des Abscheiders. Der Einbau 88 ist üblicherweise mit dem Bioreaktor verbunden, in den die eingesammelten Zellen durch das Druckgefälle oder durch Pumpen zurückgeführt werden. Vorzugsweise wird der Einbau 88 zum zentralen, vertikalen Absaugen der Feststoffe verwendet. Dies vereinfacht die Herstellung und das Platzieren des Abscheiders in seinem Behälter für den Betrieb. Auch kann der Abzug auch über eine in die Spitze des Abscheiders eingeschweißte Durchführung oder durch eine in einem Deckel oder Stopfen eingebrachte Durchführung erfolgen.

**In einer ersten Ausführungsform** der vorliegenden Erfindung besteht der Abscheidebereich aus einer Vielzahl von nebeneinander angeordneten Kanälen in einem Lamellenpaket 1, vorzugsweise hergestellt aus mehreren aufeinander gestapelten Stegplatten, die die Kanäle des Lamellenpakets 1 bilden. Bevorzugt werden Kunststoffplatten verwendet. Die Kanäle sind am unteren Ende und am oberen Ende geöffnet. Am unteren Ende führen die Kanäle zu der Einleitfläche über dem gemeinsamen nach unten, insb. konisch oder pyramidal, verjüngten Feststoffsammelbereich 57. Am oberen Ende führen sie zu einem gemeinsamen Erntestromsammelbereich 56, der mindestens eine Durchführung 80 verfügt, durch die der Erntestrom aus dem Gefäß befördert werden kann.

In den Kanälen erfolgt die Separation von Zellen und Zellkulturlösung. Durch die kontinuierliche Entnahme des Erntestroms aus dem Bioreaktor werden Zellkulturlösung und Zellen in die Kanäle eingesaugt. Im unteren Segment des mittleren Bereiches des Kunststoffbeutels oder der Kunststoffflasche werden eine oder mehrere Durchführungen oder Einbauten 84 zur gleichmäßigen horizontalen Strömungsverteilung des Reaktorgemisches 74 eingebracht. Es wird eine gleichmäßige horizontalen Strömungsverteilung der Zellkulturlösung (=Feed) 74 über eine Einleitfläche 510 angestrebt. Üblicherweise wird je nach Breite des Lamellenpakets eine bis vier Durchführungen mit geraden Ports auf gleicher Höhe im Wand Kunststoffbeutel oder der Kunststoffflasche eingebracht. Je nach Höhe und Abstand zwischen solche Durchführungen können horizontale Verteiler 85 als Einbauten vorteilhaft sein.

Die Zellen sedimentieren innerhalb der schräg angeordneten Kanäle, rutschen wie in klassischen Schrägkanalabscheidern im Gegenstrom zum zufließenden Erntestrom wieder aus den Kanälen heraus und sammeln sich in dem konisch verjüngten Feststoffsammelbereich 57. Üblicherweise verfügt der Feststoffsammelbereich 57 über einen oder mehreren Durchführungen/Einbauten 88/89, verbunden mit dem Bioreaktor zur Absaugung der eingesammelten Zellen und Rückführung in den Bioreaktor.

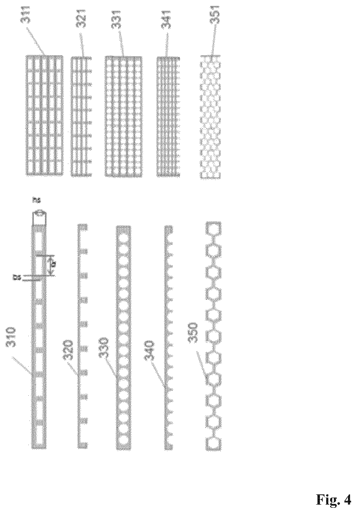

Die Kanäle des Lamellenpakets 1 können einen eckigen, elliptischen, runden oder halbrunden Querschnitt haben (Fig 4).

Die Dimensionierung der Kanäle (Anzahl, Durchmesser, Länge) hängt jeweils von der Art der zurückzuhaltenden Zellen, der Größe des Bioreaktors und dem Durchsatz ab.

Die Kanalbreite $d$ liegt bevorzugt bei $d \geq 1$ mm, um eine Verstopfung der Kanäle zu verhindern. In einer bevorzugten Ausführungsform werden Kanäle mit einer Kanalbreite von 3 mm bis 100 mm, bevorzugt von 4 mm bis 20 mm, besonders bevorzugt von 5 -7 mm eingesetzt, um einerseits Verblockungszustände sicher zu vermeiden, andererseits aber das die Raum-Zeit-Ausbeute mindernde Volumenverhältnis von Abscheider- und Bioreaktorraum möglichst gering zu halten.

Die erforderliche Abscheidefläche $A_{erf}$ ergibt sich aus der Sedimentationsgeschwindigkeit $ws$, der Perfusionsrate $q/V$ (Mediendurchsatz $q$ pro Bioreaktorvolumen $V$) und dem Bioreaktorvolumen nach Gl. 1. Ein Wirkungsgrad $\eta$ berücksichtigt die Leistungsminderung bzw. Leistungsabweichung von Schrägkanalabscheidern gegenüber Vertikalabscheidern (Gl. 2).

Die theoretische Abscheidefläche $A_{th}$ bei rechteckigen und zylindrischen Querschnitten kann nach in der Literatur (H.-J. Binder, Sedimentation aus Ein- und Mehrkornsuspensionen in schräg stehenden, laminar durchströmten Kreis- und Rechteckrohren, Dissertation Berlin, 1980) veröffentlichen Ansätzen aus den Gln. 3 und 4 näherungsweise bestimmt werden:

$$A_{erf} = \frac{Perfusionsrate \cdot V}{ws} \qquad (Gl.\ 1)$$

$$A_{th} = \frac{A_{erf}}{\eta} \qquad (Gl.\ 2)$$

$$\text{Rechteck:}\ A_{th} \approx Z \cdot \sin(\beta) \cdot d \cdot L \qquad (Gl.\ 3)$$

$$\text{Zylinder:}\ A_{th} \approx \frac{3 \cdot \pi}{16} \cdot Z \cdot \sin\beta) \cdot d \cdot L \qquad (Gl.\ 4)$$

Hierbei sind Z die Anzahl der Kanäle, $\beta$ der Winkel, um den die Kanäle gegenüber der Richtung der Schwerkraft gekippt sind, $d$ der Innendurchmesser und $L$ die Länge der Kanäle. $\pi$ ist die Kreiszahl ($\pi$ = 3,14159...).

Bei der Dimensionierung der Kanallänge ist die Einhaltung laminarer Strömungsbedingungen (Reynolds-Zahl Re < 2300) zu berücksichtigen. Die Kanallänge L richtet sich nach der Länge der zur Verfügung stehenden Beutelinnenabmessung (= Länge des Beutels LK). Die zu realisierende Länge LK des Beutels richtet sich nach dem in dem Kunststoffbeutel zu realisierenden Füllständen und nach den im Kunststoffbeutel zu realisierenden hydrostatischen Drücken. Zu große hydrostatische Drücke können ggf. an entsprechend dimensionierte, nicht produktberührte und daher wiederverwendbare Einhausungen weitergegeben werden.

Der dynamische Druck an der Erntestromabzugsstelle (=Durchführungen/Einbauten 80) sollte dabei mindestens um das 5- bis 10-fache kleiner sein als der Druckverlust in den Kanälen, um das wirkungsgradmindernde Phänomen der Maldistribution auszuschließen. Ausreichende Druckverluste sind bei Kanallängen ab 0,1 m als technisch realisierbar anzusehen, während bevorzugt Kanallängen von 0,2 m bis 5 m, besonders bevorzugt Kanallängen von 0,4 m bis 2 m realisiert werden.

Üblicherweise beträgt die Kanallängen L 30 % bis 95%, besonders bevorzugt 60% bis 90% der Länge LK des Kunststoffbeutels bzw. Kunststoffflasche.

Kurze Kanallängen L können aufgrund der reduzierten Druckverluste zu Verteilungsproblemen führen, was insbesondere bei der Abnahme des Erntestroms aus dem oberen Erntestromsammelbereich 56 eine Verteilungsvorrichtung zur Reduzierung der Abzugsgeschwindigkeiten erfordern kann. Optional weisen daher die Durchführungen/Einbauten 80 Strömungsinverter 81 für ein vergleichmäßigtes Abziehen des von den Zellen getrennten Erntestroms 70 (=Harvest) aus einem Erntestromsammelbereich 56 auf.

Der erfindungsgemäße Abscheider kann üblicherweise 1 bis 10[6] Kanäle umfassen, bevorzugt 10 bis 100.000, besonders bevorzugt 10 bis 10.000. Die Kanäle sind ggf. über eine oder mehrere Stegplatten in einem Lamellenpaket 1 zur Optimierung des Platzbedarfs verteilt. Bevorzugt umfasst das Lamellenpaket 1 von 1 bis 400 Stegplatten, besonders bevorzugt 1 bis 50 Stegplatten. In einer die Erfindung nicht einschränkenden Ausführungsform kann die Stegplatten an eine Stützplatte 30 gebunden sein, die Halt bietet und zur exakten Positionierung mit dem Kunststoffbeutel durch Kleben oder Schweißen verbunden werden kann.

Das Breiten- zu Höhenverhältnis des Lamellenpaketes 1 bestehend aus ein- oder mehrlagigen Stegplatten einschließlich der Stützplatte lässt sich an die Geometrie des Kunststoffbeutels anpassen.

In 3D-Beutel (aus 4 Folienbahnen zusammengeschweißte Beutel) lassen sich günstiger Weise Lamellenpakete 1 mit quadratischem, zylindrischem rechteckigem oder elliptischem Querschnitt mit einem Höhen- zu - Breiten-Verhältnis H/D von 0,3 < H/D < 1,5, bevorzugt 0,6< H/D < 1,2, besonders bevorzugt 0,9 < H/D < 1,0 einsetzen.

Für die einfacheren, preiswerteren 2D-Beutel (aus zwei Folienbahnen zusammengeschweißte Beutel) eignen sich flache Lamellenpakete mit rechteckigem Querschnitt bei H/D-Verhältnissen von 0,005 < H/D < 1, bevorzugt 0,02 < H/D < 0,6, besonders bevorzugt 0,04 .

Für das Verschweißen eines 2D-Beutels bei eingeschobenem Lamellenpacket ist es vorteilhaft einen Midestabstandsverhältnis $0,5 \leq X/H \leq 2$ bevorzugt $1 \leq X/H \leq 1,6$ zu halten, wobei X die Distanz zwischen dem Lamellenpaket 1 und dem Anfang einer Verjüngung und H die Dicke des Lamellenpakets ist.Das Lamellenpaket 1 kann aus einer profilierten Platte 340 oder 320 gebildet werden (siehe Figur 4). Eine profilierte Platte weist bevorzugt eine glatte Seite und eine Seite mit einer Folge von Stegen und Rinnen in gleichbleibenden Abständen auf. Kanäle bilden sich bei der Stapelung der Platte in einer oder mehreren Lagen z.B. auf eine Stützplatte 30. Dabei werden die Rinnen zur offenen Seite hin jeweils durch die glatte Seite einer benachbarten Lage bzw. durch die Wand des Stators verschlossen. Es besteht ebenfalls die Möglichkeit ein Lamellenpaket oder - teilpaket ein- oder mehrlagig zu extrudieren und zu einem Lamellenpaket 1 zu verbinden.

Die Geometrie der Kanäle wird durch das Verhältnis der Steghöhe hs zur Kanalbreite d (Fig. 4) festgelegt. Technisch realisierbare hs/d-Verhältnisse liegen je nach Beschaffenheit (Formbarkeit, Elastizität, Tiefziehvermögen) im Bereich $0{,}01 \leq hs/d \leq 5$. Üblicherweise ist hs größer als oder gleich 1 mm, bzw. bevorzugt größer als oder gleich 3 mm sein sollten. Bevorzugte hs/d-Verhältnisse liegen bei 0,5 bis 5. Die Stegbreiten bs werden durch die mechanische Stabilität des Folienmaterials bestimmt. Die Stegbreiten bs sollten möglichst klein sein, um hohe Abscheideflächen pro Abscheidervolumen zu ermöglichen. Gleichzeitig sollten sie nicht zu gering gewählt werden, um eine kraftschlüssige Verbindung mit der unteren Lage ohne Formveränderung erlauben zu können. Bei extrudierten Lamellenpaketen 1, oder bei Lamellenpaketen, die aus extrudierten Lamellenteilpaketen oder Stegplatten aufgebaut sind, können sehr hohe Steifigkeiten mit kleinen Stegbreiten ohne großen Verlust an Abscheidefläche realisiert werden, so dass diese Herstellungsform bevorzugt ist.

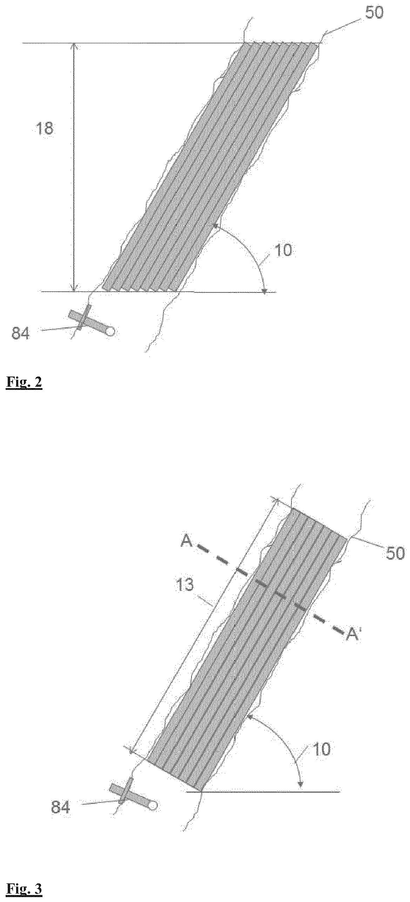

Das aus Stegplatten aufgebaute Lamellenpaket ist entweder als gerader Quader (Fig.3) ausgeführt, wobei die Ebene der Kanalöffnungen rechtwinklig zur Auflagefläche des Lamellenpaketes 1 ist, oder als schiefer Quader (Fig. 2), wobei die Kanalöffnungen im eingebauten Zustand auf einer horizontalen Ebene liegen. Letztere Lösung ist bevorzugt, um einen durch Sedimentation verursachten Konzentrationsgradienten zu den unteren Kanalöffnungen zu verhindern. Die Kanäle werden bevorzugt vom Reaktorgemisch mit Hilfe des horizontalen Verteilers (85) vergleichmäßigt angeströmt.

Eine bevorzugte Verbindung der Stegplatten erfolgt über Verklebung oder Verschweißung Das Lamellenpacket sollte durch die Verbindung in erster Linie räumlich fixiert werden. Außerdem wird angestrebt, die sog. Totzonen (nicht zur Abscheidung genutzten Räume um die Außenflächen der Stegplatten) möglichst klein zu halten. Eine vollständige Vermeidung dieser Totzonen ist dabei jedoch nicht unbedingt gefordert. Als Kleber eignen sich die dem Fachmann bekannten, auf die Material- und Oberflächeneigenschaften der Kanäle abgestimmten Klebekomponenten. Insbesondere wird bevorzugt Kleber verwendet, der in den geforderten FDA-Güteklassen im Markt zur Verfügung steht. Für das Verschweißen lassen sich thermische Verbindungstechniken wie Hitze, Laser, Ultraschall anwenden. Eine besonders bevorzugte Verbindungstechnik ist das Laserstahlschweißen, das insbesondere auch in Kombination mit dem Zuschnitt des Lamellenpaketes in einer dazu geeigneten Vorrichtung angewendet werden kann. Die Schweißtechnik besitzt den Vorteil, dass die Anzahl der in den Pharmaprozess eingeführten Kunststoffe durch diese Verbindungstechnik nicht erhöht wird.

Die profilierte Platte kann durch Formgebung unmittelbar bei der Plattenherstellung oder durch (z.B. klebetechnische) Verbindung einer geprägten, heiß oder kalt verformten Platte mit einer glatten Platte erfolgen. Die Materialeigenschaften der geprägten und glatten Platten können auf ihre unterschiedliche Funktionalität (gute Gleiteigenschaften und Formstabilität der geprägten Platten, gute Dichteigenschaften der glatten Platten) hin optimal, d.h. durch Wahl eines geeigneten, dem Fachmann bekannten Werkstoffes mit entsprechender Oberflächengüte, angepasst werden.

Üblicherweise werden kommerziell verfügbare, kostengünstige, und für Pharma-Prozesse geeignete Stegplatten insb. Kunststoffstegplatten z.B. aus Polykarbonat extrudiert und als Lamellenteilpakete zur Herstellung des Lamellenpakets 1 in der passenden Länge geschnitten bzw. hergestellt und aufeinander befestigt.

Zur Herstellung des Abscheiders werden außerdem in einer Kunststofffolie die Durchführungen und weitere Einbauten an den passenden Stellen vorbereitet und ggf. eingebaut.

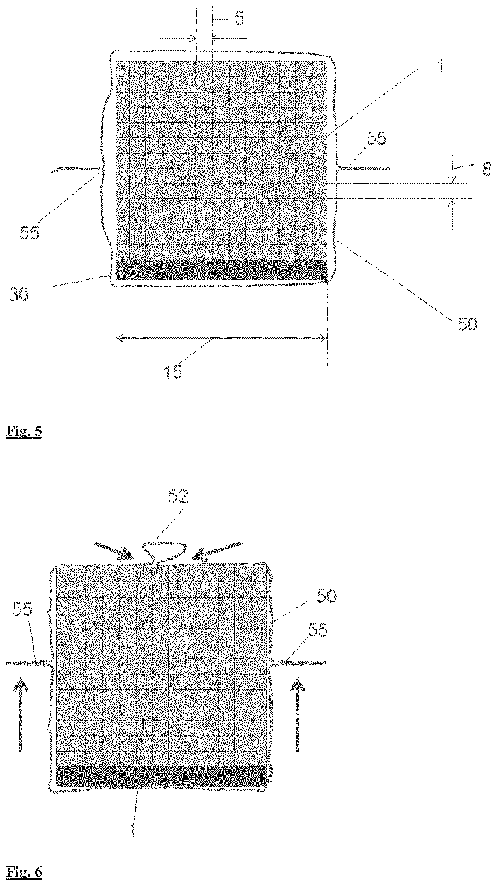

Anschließend wird ein Kunststoffbeutel 50 aus der Kunststofffolie, die das Lamellenpaket 1 einschließt, in einem Kunststoffbeutel 50 mit Schweißnaht 55 zusammengeschweißt (Fig. 5).

Das Lamellenpaket 1 einschließlich Stützplatte wird dann üblicherweise gegen die Innenflächen des Kunststoffbeutels 50 verpresst, um das Eindringen von Zellen zwischen Kunststoffbeutel 50 und Lamellenpaket 1 und damit Fouling zu verhindern.

In einer ersten Ausführungsform des Herstellungsverfahrens wird der Kunststoffbeutel 50 an das Lamellenpacket 1 gestrafft (Fig. 5) und die gebildete Falte 52 wird mittels eines oder mehrerer Befestigungsbänder 60 flach gedrückt und befestigt (Fig. 6). Als Befestigungsband eignet sich auch eine Kunststofffolie, die um Beutel und Lammellenpaket straff herumgewickelt wird. Günstige Straffungseigenschaften besitzen z.B. Haushaltsfolien ober flexible, dünne Folien aus Silikon. Auch eine Verschweißung des Lamellenpaketes 1 mit der Beutelwand kann zur Herstellung einer dichten Verbindung zwischen Beutel und Lamellenpaket geeignet sein.

Die beschriebenen Verfahren erlauben die einfache und kostengünstige Herstellung des erfindungsgemäßen Feststoffabscheiders zur Rückhaltung und Rückführung von Zellen. Durch die in weiten Grenzen variierbare Konfiguration der Lamellenpakete lässt sich die Geometrie der späteren Vorrichtung einfach und genau festlegen und im Gegensatz zu Systemen aus Edelstahl auch für sehr große Bioreaktoren bereitstellen. Die beschriebenen Verfahren erlauben insbesondere die kostengünstige Herstellung von Einwegelementen, durch deren Einsatz der Aufwand für die Bereitstellung eines nach den Pharmagrundsätzen gereinigten Rückhaltesystems auf ein Minimum reduziert werden kann.

Für den Betrieb wird die erfindungsgemäße Vorrichtung mit einem Winkel 10 = $\beta$ zur Horizontale gerichtet. Der Winkel $\beta$ richtet sich nach dem Absetz- und Abrutschverhalten der Zellen/Feststoffe und beträgt während des Betriebs $30° \leq \beta \leq 80°$ zur Horizontalen. In einer bevorzugten Ausführungsform liegt der Winkel $\beta$ von 35° bis 75°, besonders bevorzugt von 45° bis 60° zur Horizontalen.

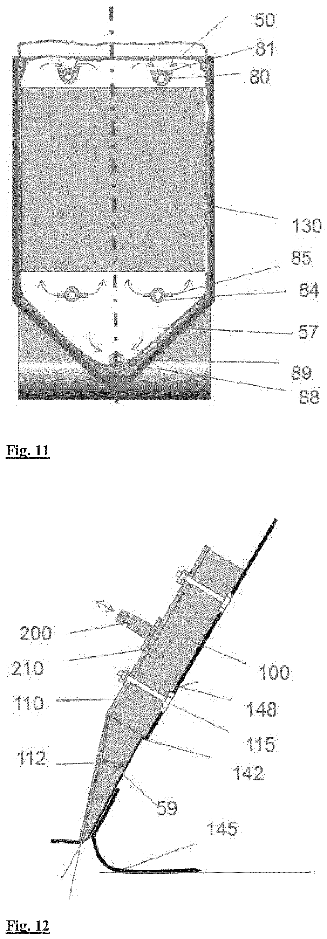

Um den Winkel $\beta$ während des Betriebs zu gewährleisten, wird der erfindungsgemäße Feststoffabscheider für den Betrieb auf einem Gestell 140 befestigt (Fig. 11 bis 13).

Üblicherweise umfasst das Gestell 140 einen Gestellfuß 145 und eine Auflage 148 mit einem vordefinierten Winkel 10 (= $\beta$) zur Stellfläche. Auf der Auflage 148 wird das Lamellenpaket 1 inkl. Stützplatte 30 mit Hilfe eines Vorsprungs 142 und / oder Deckels 110 sowie Befestigungselementen 115 auf einer vordefinierte Höhe festgehalten, damit sowohl der Erntestromsammelbereich 56 (oben) und der Feststoffsammelbereich 57 während des Betriebs möglichst faltenlos auf der Auflage ruhen können. Hiermit werden tote Räume und entsprechendes Fouling vermindert.

In einer bevorzugten Ausführungsform verfügt das Gestell 140 über ein Gehäuse 100 und einen Deckel 110 zur Aufnahme des Lamellenpakets 1.

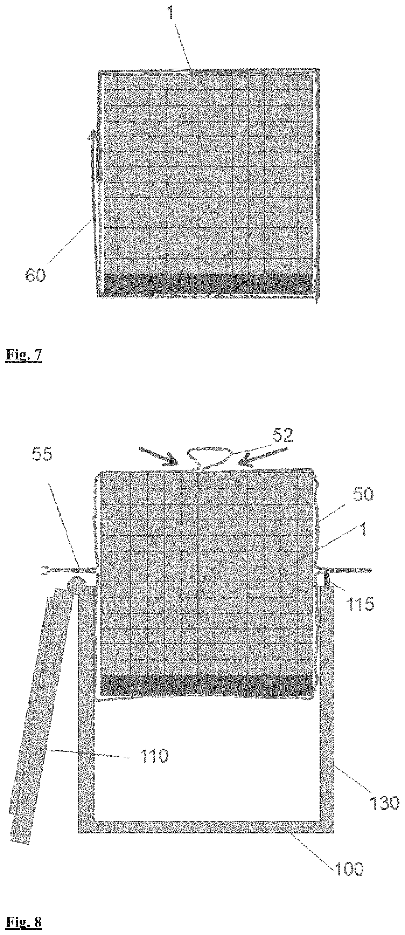

In diesem Fall kann der Straffungsprozess auch beim Einbau des erfindungsgemäßen Feststoffabscheiders auf das Gestell 140 und insbesondere in dem Gehäuse 100 und Deckel 110 (Fig. 6 und 7) ggf. auch ohne Umwicklung mit einem Befestigungsband 60 stattfinden. Hierbei wird der Kunststoffbeutel 50 mit Hilfe des Gehäuses 100 an die Stützplatte 30 und an das Lamellenpaket 1 in Position gehalten und die Falte 52 wird mit Hilfe des Deckels 110 an das Lamellenpaket 1 angepresst. Vorzugweise ist der Deckel 110 an dem Gehäuse 130 an einer Seite z.B. mittels Scharnieren und an der anderen Seite mittels einem oder mehreren verschließbaren Befestigungselementen 115 befestigt. Hiermit ist das Gestell 140 für die Inbetriebnahme des erfindungsgemäßen Feststoffabscheiders einfacher zu betätigen.

In einer bevorzugten Ausführungsform verfügt der Deckel 110 über eine Verlängerung 112 und / oder einen Rahmen 130, die den nach unten verjüngten Feststoffsammelbereich 57 in Form, insbesondere den Winkel 59 konstant, hält und dessen Ausdehnen im gefüllten Zustand während des Betriebs verhindern. Ein solcher formpassender Behälter ist u. a. vorteilhaft für den Betrieb des Systems bei größeren hydrostatischen Kräften, wie sie beim Anschluss an große Bioreaktoren zu erwarten sind.

Zur Verbesserung des Abrutschverhaltens der Zellen in den Kanälen des Lamellenpakets und auf den Innenwänden des nach unten, insb. konisch oder pyramidal, verjüngten Feststoffsammelbereichs kann die Vorrichtung mit geeigneten Mitteln, beispielsweise pneumatischen oder elektrischen Vibratoren, zur Vibration gebracht werden.

Die erforderlichen Abscheideflächen richten sich nach den Sedimentationseigenschaften der Zellen sowie den angestrebten Perfusionsraten und Zellkonzentrationen. Bevorzugte Perfusionsraten liegen im Bereich von 0,1 bis 40 1/Tag, besonders bevorzugte von 0,5 bis 20 1/Tag. Bevorzugte Abscheideflächen pro Bioreaktorvolumen liegen je nach Sedimentationseigenschaften der Zellen (abhängig von der Konzentration, Größe und Agglomerationsneigung der Zellen) im Bereich von 0,1 bis 100 $m^2/m^3$ besonders bevorzugte von 2 bis 20 $m^2/m^3$.

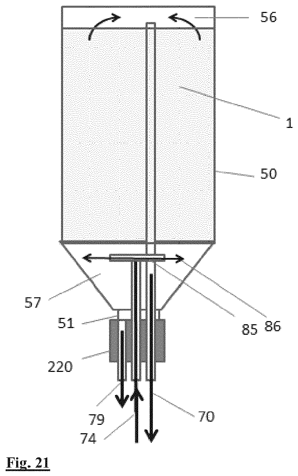

Alternativ zu dem Kunststoffbeutel kann ein Lamellenpaket 1 in einer Kunststoffflasche 50 mit eckigem Querschnitt eingebaut sein (Fig. 21), wobei die Kunststoffflasche einen nach unten verjüngten Bereich aufweist, der in einem Flaschenhals endet und, wobei der nach unten verjüngten Bereich den Feststoffsammelbereich 57 zum Sammeln der Feststoffe mit Hilfe der Schwerkraft bildet. Für den Betrieb wird der Kunststoffflaschenhals mit Hilfe eines Deckels 220, der die Durchführungen für das Hindurchtreten der Anschlussleitungen aufweist, verschlossen. In einer Alternativausführungsform wird das Lamellenpaket 1 durch einen oder mehrere an die Wände der Kunststoffflasche 50 befestigten Böden als Abscheidebereich ersetzt.

Als Material für die Kunststoffflasche eignen sich z. B. das Material aus den kommerziellen erhältlichen Millicell® Kulturflaschen der Firma Merck Millipore von (http://www.millipore.com/catalogue/module/c85149).

Der erfindungsgemäße Feststoffabscheider ist zur Vermeidung der Reinigungsproblematik bevorzugt als Einwegartikel ausgeführt.

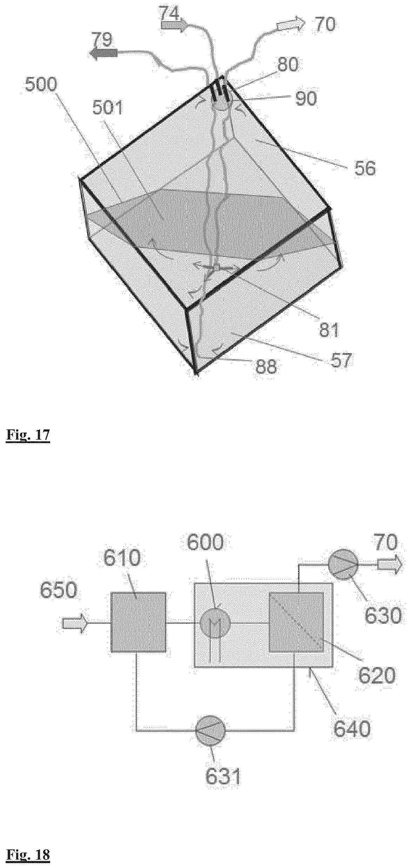

Üblicherweise wird der erfindungsgemäße Zellenabscheider an einem Bioreaktor z. B. an einem Einweg Bioreaktor wie in US 2009-0180933 beschrieben extern mittels Schlauchleitungen angekoppelt. Die Versorgung des erfindungsgemäßen Abscheiders wird durch mindestens zwei Pumpen, bevorzugt scherarmen Schlauchpumpen, sichergestellt (Fig. 18). Die Pumpen ermöglichen die Entnahme der Zellkulturlösung aus dem Bioreaktorraum, deren Zuleitung nach Kühlung über einen Wärmeaustauscher zur Abscheidevorrichtung, die Entnahme des Erntestroms aus der Abscheidevorrichtung und den Rücktransport des Feststoffstroms (=Return 70) zum Bioreaktor.

Die Lagerung der erfindungsgemäßen Feststoffabscheider ist platzsparend, da sie problemlos aufeinander gestapelt werden können und erst bei der Inbetriebnahme mit dem passenden Winkel aufgestellt werden. Sie lassen sich dann einfach außerhalb eines Bioreaktors anschließen und betreiben. Der Anschluss an die Fermenter erfolgt mittels am Ende der Schlauchleitungen befestigter Steril-Kuppler diverser Hersteller (Fa. Pall, Fa. Sartorius, Fa. Coulder) innerhalb oder außerhalb von Sicherheitswerkbänken, vorzugsweise jedoch durch Schlauchschweißen. Die an den erfindungsgemäßen Feststoffabscheidern befestigten Schlauchleitungen sind daher bevorzugt - zumindest teilweise - mit einem das Schlauchschweißen geeigneten Schlauchelement ausgestattet. Außerdem enthalten üblicherweise die Schlauchleitungen zur Förderung der Suspension mindestens zwei spezielle mechanisch hoch belastbare Schlauchelemente (z.B. aus Elastomerschlauch Verderprene der Fa. Verder), die nicht-invasiv in Schlauchpumpen eingelegt werden können, ohne die Sterilität der Abscheider zu gefährden. Der Anschluss, der Betrieb und die Wartung sind problemlos. Die Ausführung der erfindungsgemäßen Vorrichtung oder Teile der erfindungsgemäßen Vorrichtung als Einwegelement eliminiert Reinigungsprobleme.

Weitere Gegenstände der vorliegenden Erfindung sind daher ein Verfahren zur Herstellung einer erfindungsgemäßen Feststoffabscheidevorrichtung mit Lamellenpaket in einem Kunststoffbeutel sowie

Ein direkter Einsatz des Lamellenpakets 1 in aerobe Bioreaktoren ist prinzipiell denkbar, wenn die zur Begasung erforderlichen Gasblasen von den Zutrittsöffnungen ferngehalten werden können. In diesem Fall können der konische Sammelteil des Kunststoffbeutels sowie die Rücklaufpumpe entfallen.
Bevorzugt ist der erfindungsgemäßen Abscheider jedoch für die Verwendung außerhalb eines Bioreaktors vorgesehen.

**In einer zweiten Ausführungsform** des erfindungsgemäßen Zellenabscheiders ist der Kunststoffbeutel polyeder- oder kegelförmig, wobei der Kunststoffbeutel während des Betriebs so gestellt ist, dass der unten konisch verjüngte Feststoffsammelbereich 57 durch die Wände des Kunststoffbeutels und eine Spitze bzw. Ecke des Polyeders oder der Kegels gebildet wird. Innerhalb des Kunststoffbeutels beträgt der Winkel 10 der Abscheidefläche 500 0° zur Horizontalen, wie bei herkömmlichen Vertikalabscheidern.

Das Volumen des Abscheiders kann beispielsweise Werte von 0,1 L bis 1000 L annehmen.

Insbesondere wird die polyedrische Form des erfindungsgemäßen Abscheiders ausgewählt aus der Gruppe bestehend aus einem Disphenoid (=ein von vier deckungsgleichen Dreiecken begrenzter Polyeder, wie in Fig. 14 und 15 dargestellt), insbesondere Tetraeder, gerade Pyramide (=ein von vier deckungsgleichen Dreiecken und einem Quadrat begrenzter Polyeder, nicht abgebildet), Oktaeder und Würfel (Fig. 16 und 17 dargestellt).

Ebenfalls geeignet sind Kegel (= zusammengesetzt aus dem Kegelmantel und einer Ronde).

Für eine einfache Herstellung aus einer Kunststofffolie werden Disphenoid insb. Tetraeder, Kegel und Würfel bevorzugt. Der erfindungsgemäße Zellenabscheider weist üblicherweise ein Verhältnis von Höhe zur maximalen Breite im Bereich von 0,2 bis 3, bevorzugt 0,5 bis 2, besonders bevorzugt 0,7 bis 1,5 auf.

Die Verbindung des erfindungsgemäßen Zellabscheiders mit einem Bioreaktor oder anderen externen Anlagen erfolgt bevorzugt über Anschlüsse und Verbindungsleitungen am Kopf des Abscheiders. Das hat den Vorteil, dass der Behälter zur Aufnahme der Abscheidereinrichtung ohne Durchführung, d. h. leckagefrei, ausgeführt werden kann. Hierdurch lässt sich ohne zusätzliche Sicherheitseinrichtungen das Austreten von gentechnisch veränderten Produktionszellen verhindern.

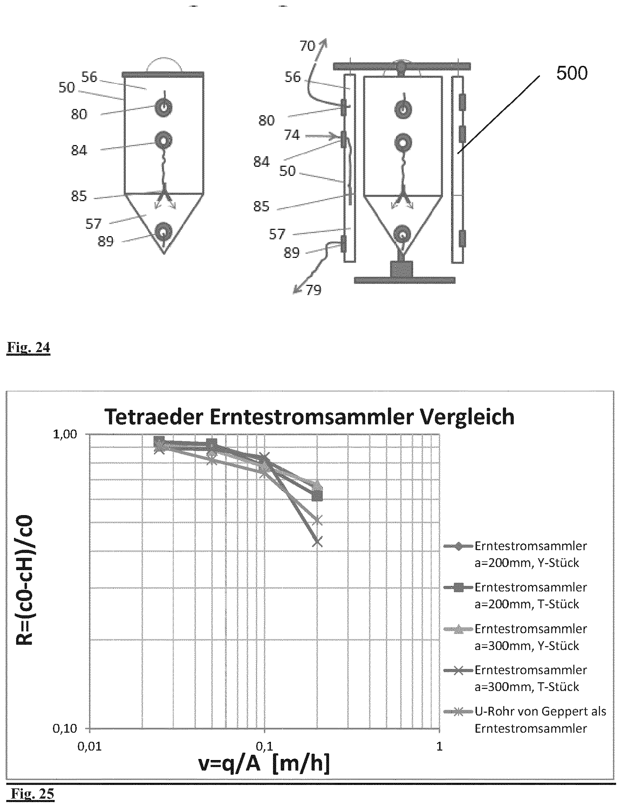

Der Einlass des Bioreaktorgemisches erfolgt üblicherweise über einen Einbau 84 zur Einführung des Bioreaktorgemisches entlang der Vertikalachse des für den Betrieb aufgestellten Kunststoffbeutels. Bevorzugt werden bei dem erfindungsgemäßen Zellenabscheider an der Einlassöffnung des Einbauelementes 84 horizontale Verteiler 85 zur gleichmäßigen horizontalen Strömungsverteilung des Reaktorgemisches 74 verwendet. Der Zulauf wird dabei mittels ringförmiger Düsen oder mittels zweier oder mehrerer Austrittsöffnungen horizontal gerichteten Einbauten wie z. B. T-Stücke oder nach unten gerichteten Einbauten wie z. B. Y-Stücke über Einleitfläche 510 bevorzugt in Richtung der der Ecken der Einleitfläche 510 verteilt. Fig. 25 zeigt einen Vergleich zwischen Einbau 84 zur Einführung des Bioreaktorgemisches mit einem einfachen T-oder Y-Verteiler sowie mit doppelten T-oder Y-Verteilern. Bei den Y-Verteilern werden die Einlaufströme nach unten gerichtet. Der Einfluss des Verteilers bei kleiner Klärflächenbelastung v ≤ 0.1 m/h ist gering. Eine bessere Rückhaltung wurde mit einem einfachen T-oder Y-Verteiler als mit doppelten T-oder Y-Verteilern erreicht. Bei hohen Flächenbelastungen v ≥ 0.1 m/h sind die Y-Verteiler mit den nach unten gerichteten Einlaufströme den T-Verteilern überlegen und entsprechend bevorzugt.

Üblicherweise werden Verteiler mit von 2 bis 8 bevorzugt 2 bis 4 horizontal oder nach unten gerichteten Öffnungen verwendet, wobei eine Breite c (=Abstand zwischen zwei Öffnungen) zur Kantenlänge D des Kunststoffbeutels $0,03 \leq c/D \leq 0,25$ bevorzugt $0,04 \leq c/D \leq 0,15$ üblicherweise verwendet wird. Im Falle eines kegelförmigen Abscheiders ist D der Durchmesser der Ronde.

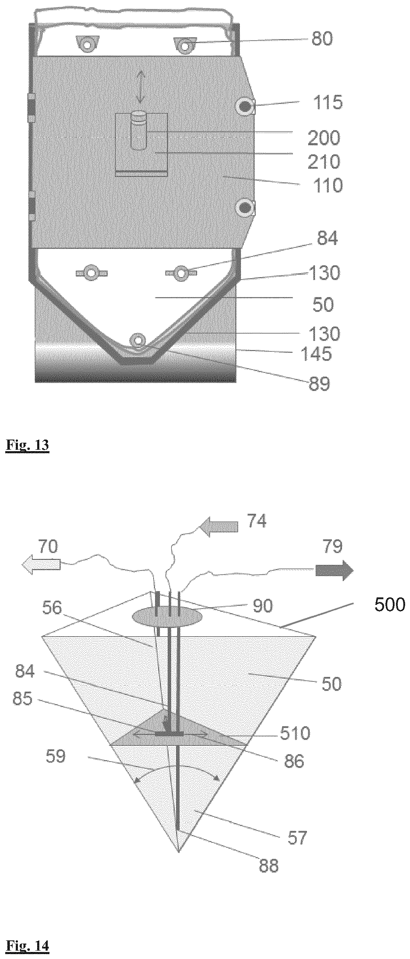

Das Abziehen der Feststoffe aus dem Feststoffsammelbereich 57 erfolgt üblicherweise über eine oder mehrere Durchführungen bzw. Einbauten 88 in der Nähe der unteren Spitze des Abscheiders. Der Einbau 88 ist verbunden mit dem Bioreaktor, in den die eingesammelten Zellen durch das Druckgefälle oder durch Pumpen zurückgeführt werden. Vorzugsweise wird der Einbau 88 zum zentralen, vertikalen Absaugen der Feststoffe verwendet (Fig. 14, 15, 16, 17). Dies vereinfacht die Herstellung und das Platzieren des Abscheiders in seinem Behälter für den Betrieb. In einer weiteren bevorzugten Ausführungsform kann der Abzug auch über eine in die Spitze des Abscheiders eingeschweißte Durchführung abgezogen werden. Für eine leckagefreie Ausführung des Gehäuses ist in diesem Fall der Anschlussschlauch im Inneren des Gehäuses zur Aufnahme des Abscheiders (d.h. zwischen Gehäusewand und Kunststoffbeutel) zu verlegen.

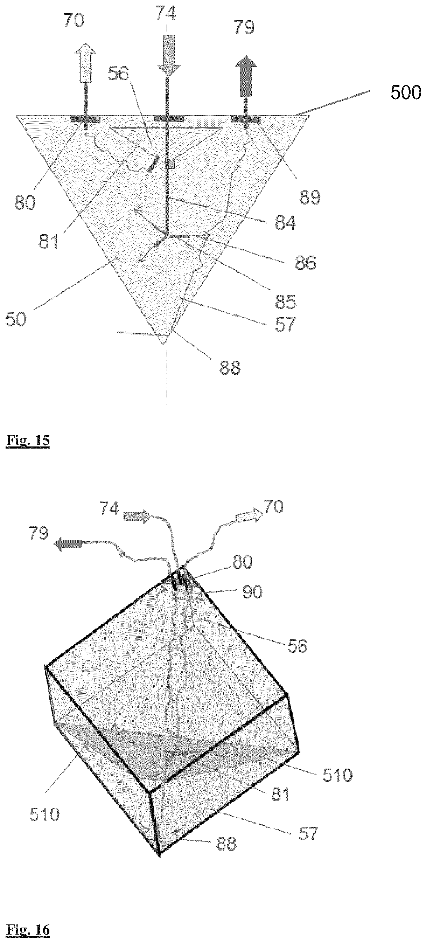

Das Abziehen des Erntestroms erfolgt üblicherweise über einen Einbau 80, der sich innerhalb des Kunststoffbeutels im Erntestromsammelbereich 56 oberhalb der Abscheidefläche 500 befindet. In einer bevorzugten Ausführungsform wird im Erntestromsammelbereich 56 ein Strömungsinverter 81 für ein vergleichmäßigtes Abziehen des von den Zellen getrennten Erntestrom 70 (=Harvest) aus dem Erntestromsammelbereich 56 eingebaut, der mit dem Einbau 80 verbunden ist (Fig. 15). Dieser Einbau 80 weist üblicherweise die Form eines invertierten Schirms mit dem Außenkontur des Erntestromsammelbereichs 56 (im Falle eines Tetraeders ist wird eine dreieckigen Einbau verwendet), mit einer Kantenlänge bzw. Durchmesser a auf, wobei $0,25 \leq a/D \leq 0,75$ bevorzugt $0,4 \leq a/D \leq 0,6$, besonders bevorzugt 0,5 sein kann.

Üblicherweise sind die Einbauten 80, 88 und 84 eingeschweißte Durchführungen, an die Leitungen, vorzugsweise Schläuche zur Verbindung mit dem Bioreaktor oder anderen externen Anlagen, angeschlossen werden können.

In einer bevorzugten Ausführungsform des erfindungsgemäßen Abscheiders befindet sich im oberen Bereich an der Wand des Kunststoffbeutels (Tetraeder) oder an einer Ecke (Würfel) eine oder mehrere Anschlussplatten 90, vorzugsweise eine, die auch ein Deckel sein kann, die die Durchführungen für das Hindurchtreten der Anschlussleitungen enthält und im Bereich des Anschlusses eine Halterung des Abscheiders ermöglicht. Sie wird üblicherweise bei der Montage mit dem Gehäuse des Abscheiders verbunden.

Die maximale Abscheidefläche 500 befindet sich oberhalb der Einlassfläche 510. Die Separation der Zellen erfolgt bei den vertikalen Abscheidern gemäß Fig. 14 bis 17 in dem sich konisch nach oben bis zum Maximalquerschnitt der Abscheidefläche 510 erweiternden Behälterquerschnitt gemäß der positiven Kräftedifferenz zwischen Schwerkraft und vertikaler Anströmung infolge kontinuierlicher Entnahme des Erntestroms aus dem Erntestromsammelbereich 56.

Die Dimensionierung der Sedimentationsabscheider hängt von der Art der zurückzuhaltenden Zellen, der Größe des Bioreaktors und dem Durchsatz ab.

Die erforderliche Abscheidefläche $A_{erf}$ ergibt sich wie in der ersten Ausführungsform der Erfindung aus der Sedimentationsgeschwindigkeit *ws*, der Perfusionsrate *q/V* (Mediendurchsatz q pro Bioreaktorvolumen *V*) und dem Bioreaktorvolumen nach Gl. 1. Ein Wirkungsgrad $\eta$ berücksichtigt die Leistungsminderung technischer Abscheider gegenüber der idealisierten Betrachtung eines von unten angeströmten Einzelpartikels, dessen Sinkgeschwindigkeit ws um die Bedingung für die Abscheidung zu erfüllen ein bisschen größer sein muss als die Anströmgeschwindigkeit = Perfusionsrate x V / Aerf (Gl. 2).

Wie bereits aufgeführt:

$$A_{erf} = \frac{Perfusionsrate \cdot V}{ws} \qquad (\text{Gl. 1})$$

$$A_{th} = \frac{A_{erf}}{\eta} \qquad (\text{Gl. 2})$$

Die Bestimmung der theoretischen Abscheidefläche für polyedrische Kunststoffbeutelabscheider wird aus Gl. 5 oder Gl. 6 näherungsweise bestimmt:

Für Tetraeder mit der Kantenlänge D gilt für die Bestimmung der maximalen Abscheidefläche in erster Näherung:

$$A_{th} = \frac{1}{4} D^2 \sqrt{3} \qquad (\text{Gl.5})$$

Weil die Abzugsstelle konstruktiv etwas unterhalb des maximalen, durch die Kantenläge des Tetraeders gegebenen Querschnitts liegt, ist der Wirkungsgrad $\eta$ etwas kleiner als 1.

Das Gleiche gilt für kegelförmige Abscheider, deren maximale Abscheiderfläche sich aus dem Rondendurchmesser D ergibt:

$$A_{th} = \frac{\pi}{4} D^2 \qquad (\text{Gl.6})$$

Für Würfel mit der Kantenlänge D ergibt sich als maximale Abscheidefläche bei $\eta$ = 1 ein Sechseck mit folgener Querschnittfläche:

$$A_{th} = \frac{3}{4} D^2 \sqrt{3} \qquad (\text{Gl.7})$$

Bei der Dimensionierung der zweiten Ausführungsform des erfindungsgemäßen Abscheiders ist die Einhaltung laminarer Strömungsbedingungen ebenfalls zu berücksichtigen. Bei der Dimensionierung des Kunststoffbeutels (50) richtet sich die zu realisierende Kantenlänge D bzw. die Länge LK der Beutel nach den in dem Kunststoffbeutel zu realisierenden Füllständen. Zu große hydrostatische Drücke können ggf. an entsprechend dimensionierte, nicht produktberührte und daher wiederverwendbare Einhausungen weitergegeben werden.

Üblicherweise befindet sich die Einlassfläche 510 in einer Höhe h bezogen auf die Höhe des Kunststoffbeutels HK von $0.3 \leq h/HK \leq 0.7$ bevorzugt von 0.4 bis 0.6. Fig. 26 zeigt den Einfluss der Höhe der Einlassfläche auf die Rückhaltung in einem Würfelabscheider.

Zur bestimmungsgemäßen Verwendung wird der Abscheider in einen Behälter eingebracht, der die flexiblen Wände des Kunststoffbeutels stützt, wenn der Abscheider gefüllt ist. Daher sind die Formen von Behälter und Abscheider vorzugsweise aufeinander abgestimmt.

Weiterer Gegenstand der vorliegenden Erfindung ist daher eine Feststoffabscheidevorrichtung umfassend einen erfindungsgemäßen Feststoffabscheider und einen Behälter zur Aufnahme des Feststoffabscheiders, wobei der Behälter mindestens:

- einen Innenraum zur Aufnahme des Feststoffabscheiders, wobei der Innenraum an die Form der Feststoffabscheiders mittels an die Form des Feststoffabscheider angepasste Wände, die den Innenraum einschließen und von der Außenwelt abgrenzen, angepasst ist,

- eine Öffnung zur Einführung der Feststoffabscheiders von oben in den Behälter,

umfasst.

Vorzugsweise insbesondere für die zweite Ausführungsform der Abscheidevorrichtung weist der Behälter einen Kanal auf, über den Schläuche und/oder Kanäle und/oder Messsonden an die Zellenabscheidevorrichtung herangeführt werden können.

Die Öffnung zur Einführung der Zellenabscheidevorrichtung ist bevorzugt schließbar.

Der erfindungsgemäße Behälter ist im geschlossenen Zustand vorzugsweise flüssigkeitsdicht ausgeführt, d.h. er kann gegenüber der Außenwelt so abgedichtet werden, dass keine Flüssigkeit ungewollt aus dem Inneren des Behälters in die Außenwelt gelangt.

Auch möglich ist ein Kunststoffbeutel oder eine Kunststoffflasche mit einem viereckigen Querschnitt und im unteren Bereich einem nach unten verjüngten Feststoffsammelbereich 57 zum Sammeln der Feststoffe mit Hilfe der Schwerkraft bildet (Fig. 24). Einbauten und Durchführungen 80, 88 und 84 können in dem Kunststoffbeutel bzw. Kunststoffflasche eingeschweißt sein. Alternativ endet der nach unten verjüngte Feststoffsammelbereich 57 in einem Hals. Sowohl der Kunststoffbeutelhals als der Kunststoffflaschenhals wird dann mit einem Deckel oder Stopfen verschlossen, in dem alle nötigen Durchführungen eingebaut sind. Bei beiden Ausführungsformen kann die Form des Erntestromsammelbereichs 56 beliebig sein. Diese Kunststoffbeutel bzw. - flaschen sind für kleine Fermentervolumen olumen von 0,02 bis 2 L besonders vorteilhaft. Für den Betrieb werden ein oder mehrere Kunststoffbeutel bzw. -flaschen an einem Gestell aufgehängt. Für eine platzsparende Anwendung mehrerer Abscheider in Parallel sind die hängenden Kunststoffbeutel bzw. Kunststoffflasche aus Fig. 24 vorteilhaft.

Weiterer Gegenstand der vorliegenden Erfindung ist eine Bioreaktoranlage bestehend aus einem Bioreaktor und einer der beschriebenen erfindungsgemäßen Zellenabscheidevorrichtung. Bevorzugt ist der Bioreaktor ein Einweg-Reaktor, insbesondere ein wie in US 2009-0180933 beschriebener Reaktor.

Die Bioreaktoranlage ist beispielsweise ein Perfusionsreaktor, der in bekannter Weise betrieben werden kann. Nährmedium wird in den Bioreaktor kontinuierlich zugeführt, und zellarmer Zellkulturüberstand wird kontinuierlich abgeführt. Der Perfusionsreaktor kann bei hohen Perfusionsraten $q/V$ (Mediendurchsatz $q$ pro Bioreaktorvolumen $V$) betrieben werden, wenn dies biologisch sinnvoll ist und eine ausreichende Abscheidefläche zur Verfügung gestellt wird. In diesem Fall erfolgt die Durchströmung des Abscheiders kontinuierlich.

Ebenso kann der Perfusionsreaktor derart betrieben werden, dass man eine Kultur zunächst absatzweise hochwachsen lässt. Wenn das Medium so weit verbraucht ist, dass kein nennenswerter Aufbau von Biomasse mehr möglich ist, zieht man über den externen Zellenabscheider Kulturüberstand ab, der nahezu frei von Biomasse ist. Den im Bioreaktor gewonnen Raum kann man dann nutzen, um frisches Nährmedium zuzuführen, wodurch weiteres Wachstum und damit eine höhere Gesamtbiomasseproduktivität ermöglicht werden (Repeated-Batch-Modus). In diesem Fall erfolgt die Durchströmung des Zellenabscheiders absatzweise. Dieses Verfahren bietet sich zum Beispiel für Vorkulturen an, mit denen sehr große Bioreaktoren geimpft werden sollen, da es die Produktivität bestehender Vorkulturreaktoren erhöhen kann.

Für den Betrieb an Bioreaktoren wird eine kontinuierliche Strömung des erfindungsgemäßen Zellenabscheiders bevorzugt.

Der Bioreaktor oder Perfusionsreaktor kann zur Züchtung von Zellen eingesetzt werden, die in vitro und in freier Suspension oder auf Mikroträger wachsen. Zu den bevorzugten Zellen, gehören Protozoen sowie adhäsive und nicht-adhäsive Eukaryoten Zellen menschlichen (Nerven-, Blut- oder Gewebezellen, sowie Stammzellen embryonaler oder adulter Herkunft), tierischen oder pflanzlichen Ursprungs, die z.B. durch gentechnische Veränderung befähigt sind, spezielle pharmazeutische Wirkstoffe wie Viren, Proteine, Enzyme, Antikörper, Neuronen, Gewebezellen oder diagnostische Strukturen zu produzieren. Besonders bevorzugt werden für die pharmazeutische Hochleistungsproduktion geeignete Zellen verwendet, zum Beispiel Ciliaten, Insektenzellen, Baby Hamster Kidney (BHK) Zellen, Chinese Hamster Ovary (CHO) Zellen, HKB-Zellen (durch die Fusion von humaner HEK 293-Zelllinie mit der humanen Burkitt Lyphomzelllinie 2B8 entstanden), Hybridoma Zellen sowie Stammzellen.

In einer alternativen Ausführungsform der Anlage ist einer der beschriebenen erfindungsgemäßen Zellenabscheider im absatzweise durchgeführten Betrieb nach Beendigung der Fermentation vor der Zellabtrennung an einen weiteren Bioreaktor oder einen Erntetank angeschlossen mit dem Ziel, die auf die Filter aufzutragende Zellmasse und damit die erforderlichen Filterflächen zu reduzieren.

Gegenstand der vorliegenden Erfindung ist weiterhin ein Verfahren zur Rückhaltung und Rückführung von Feststoffen, insbesondere Zellen, in einem durchströmten Gefäß, wobei dem Gefäß kontinuierlich oder absatzweise feststoffenthaltendes Medium zugeführt und feststofffreies Medium abgeführt wird, dadurch gekennzeichnet, dass das Gefäß ein durchströmter gammasterilisierbarer Kunststoffbeutel oder -flasche ist, der im unteren Bereich schräg angestellte Flächen, günstiger Weise einen unten, insb. konisch oder pyramidal, verjüngten Feststoffsammelbereich 57 zum Sammeln der Feststoffe mit Hilfe der Schwerkraft aufweist. In einer besonderen Ausführungsform des Verfahrens wird die Abscheidefläche durch schräg gestellten Kanälen gebildet und es herrscht bevorzugt eine Strömungsgeschwindigkeit, welche die Wahrung laminarer Strömungszustände gemäß Re < 2300 gestattet, womit eine effizienzmindernde Resuspendierung der abgeschiedenen Zellen gegen das Erdschwerefeld vermieden wird.

Die Reynolds-Zahl Re kann nach Gl. 7 aus der über den Querschnitt gemittelten Strömungsgeschwindigkeit w, der kinematischen Viskosität v des strömenden Mediums und dem Innendurchmesser d eines Kanals berechnet werden:

$$\text{Re} = (w \cdot d / v) \qquad \text{(Gl. 7)}$$

In schrägen Kanälen herrscht an den Kanalinnenwänden eine geringere Strömungsgeschwindigkeit als in den Kanalmitten. Die Zellen sedimentieren in den Kanälen und rutschen an der Unterseite der Kanäle entgegen der Strömungsrichtung den unteren Kanalenden entgegen. Die von den Zellen befreite Zellkulturlösung wird durch die Kanäle in einen Erntestromsammelbereich 56 abgegeben, der oberhalb der Kanäle angeordnet ist, und schließlich aus dem Gefäß befördert.

In einem polyeder- bzw. kegelförmigen Abscheider ist d die Breite bzw. der Durchmesser des Querschnitts der maximalen Abscheidefläche. An den Kunststoffwänden herrscht eine geringere Strömungsgeschwindigkeit als in den Kanalmitten. Die Zellen rutschen entgegen der Strömungsrichtung dem Feststoffsammelbereich 57 entgegen. Die von den Zellen befreite Zellkulturlösung steigt in einen Erntestromsammelbereich 56 auf, der oberhalb der Abscheidefläche angeordnet ist, und wird schließlich aus dem Gefäß befördert.

Das erfindungsgemäße Verfahren kann vorzugsweise außerhalb eines Bioreaktors ausgeführt werden. Hierzu wird die Zellkulturlösung mit Zellen aus dem Bioreaktor in den erfindungsgemäßen Zellenabscheider befördert. Bevorzugt werden die Zellen vor Eintritt in den Abscheider in einem externen Gefäß gekühlt, um den Stoffwechsel zu verlangsamen und somit einer produktivitätsmindernden Unterversorgung der Zellen entgegenzuwirken. In gekühlter Suspension ist eine Sauerstoffversorgung der sedimentierenden Zellen nicht erforderlich. Zumeist ist eine Abkühlung der Zellkulturlösung auf die Umgebungstemperatur der Abscheider völlig ausreichend, so dass neben dem gewünschten Stoffwechseleffekt Konvektionsströmungen sicher vermieden werden. Zur Überwachung der ausreichenden Versorgung der Zellen kann der Abscheider mit mindestens einem Einwegsensor z:B. zur Messung der Sauerstoffkonzentration und/oder des pH-Wertes ausgestattet werden. Eine Unterbringung der Sensoren ist sowohl in den Wänden wie auch den Verbindungsleitung zum Bioreaktor oder den Erntegefäßen möglich.

Das erfindungsgemäße Verfahren erlaubt die effektive Rückhaltung und Rückführung von Zellen in einem kontinuierlich durchströmten sterilen Kunststoffbeutel. Bei der Rückhaltung und Rückführung wirken auf die Zellen nur moderate Scherkräfte, die zumeist von den Zellen gut vertragen werden. Die Zellen werden in der Abscheidevorrichtung auf Fermentationstemperatur oder einem erniedrigten Temperaturniveau gehalten und die Versorgung mit Nährstoffen ist gegeben.

Im Folgenden werden Ausführungsbeispiele der Erfindung anhand von Zeichnungen näher erläutert ohne die Erfindung hierauf zu beschränken.

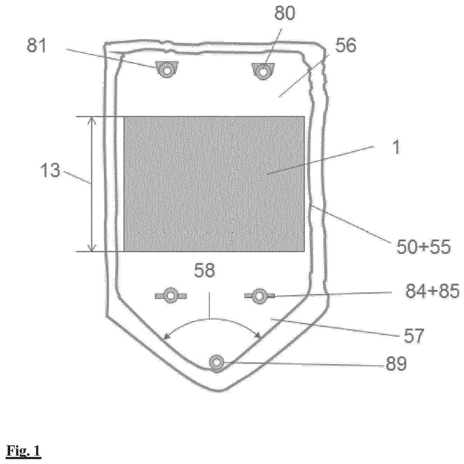

Fig 1. Schematische Darstellung der erfindungsgemäßen Einweg-Feststoffabscheider mit Lamellenpaket.

Fig. 2 Schematische Darstellung eines Lamellenpakets 1 (Längsschnitt)

Fig. 3 Schematische Darstellung eines Lamellenpakets 1 (Längsschnitt)

Fig. 4 Schema des Aufbaus verschiedener Lamellenpakete (Querschnitt AA' von Fig. 3)

Fig. 5 Schema des Aufbringens des Kunststoffbeutels 50 auf einem Lamellenpaket 1 (Querschnitt AA' von Fig. 3)

Fig. 6 und Fig. 7 Straffung und Befestigung des Kunststoffbeutels 50 auf einem Lamellenpaket 1 (Querschnitt)

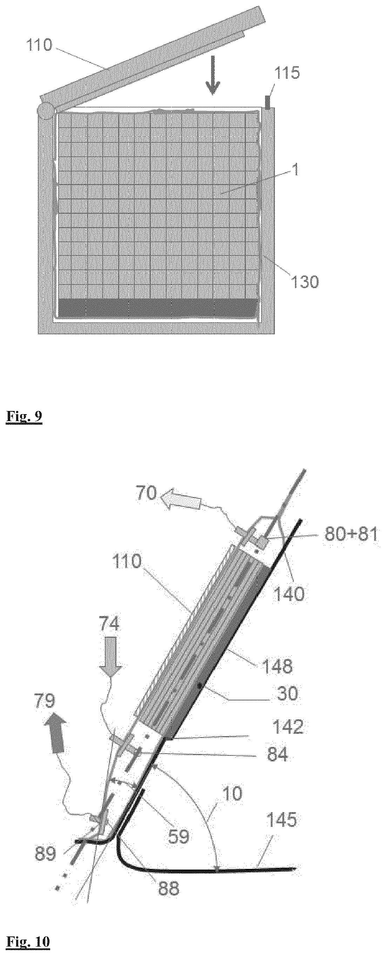

Fig. 8 und Fig. 9 Alternative Straffung und Befestigung des Kunststoffbeutels 50 auf einem Lamellenpaket 1 mit Hilfe von Rahmen 130 und Deckel 110 (Querschnitt)

Fig. 10 Seitenansichten der erfindungsgemäßen Feststoffabscheider mit Lamellenpaket 1 auf Gestell 140.

Fig. 11 Frontansichten der erfindungsgemäßen Feststoffabscheider mit Lamellenpaket 1 auf Gestell 140.

Fig. 12 Längsschnitte der erfindungsgemäßen Feststoffabscheider mit Lamellenpaket 1 auf Gestell 140 mit Rahmen 130 und Deckel 110.

Fig. 13 Frontansichten der erfindungsgemäßen Feststoffabscheider mit Lamellenpaket 1 auf ihrem Gestell 140 mit Rahmen 130 und Deckel 110.

Fig 14. Schematische 3-dimensionale Darstellung der erfindungsgemäßen Feststoffabscheider in der disphenoidischen Ausführungsform

Fig 15. Schematischer Längsschnitt der erfindungsgemäßen Feststoffabscheider in der disphenoidischen Ausführungsform mit Strömungsinverter 81

Fig. 16 und 17 Schematische Darstellung der erfindungsgemäßen Feststoffabscheider in der kubischen Ausführungsform.

Fig. 18 Verfahrensschema eines Perfusionsreaktors. Um die respiratorische Aktivität der Zellen im Bioreaktorablauf zu reduzieren, wird dessen Temperatur möglichst unmittelbar nach dem Abzug in einer Kühlvorrichtung auf ein niedrigeres Niveau abgesenkt. Auf diese Weise wird verhindert, dass die Zellen in der Zellenabscheider zu lange in einem Sauerstoff-limitierten Zustand verweilen, was die Zellen physiologisch beschädigen könnte. Im gezeigten Bespiel besteht der Abscheider 640 aus einem Abscheidebeutel 620 und einer integrierten Kühlvorrichtung 600. Die Flüssigkeitsströme zwischen Bioreaktor 610 und Abscheider 640 werden durch die scherarmen Pumpen 630 und 631 eingestellt. Andere Verschaltungen, z.B. die Positionierung von einer der beiden Pumpen 630 und 631 im Bioreaktorablauf, sind ebenso denkbar.

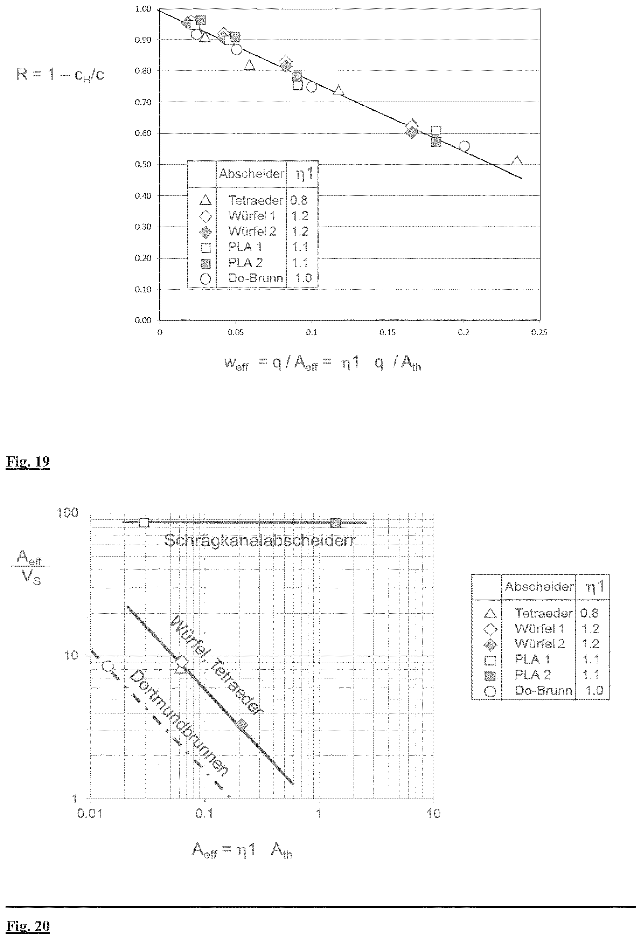

Fig.19 Vergleich der Abscheidesysteme

Fig.20 Vergleich der Abscheidervolumina pro Abscheidefläche.

Fig. 21 Längsschnitte des Flaschenabscheiders mit Böden als Abscheidefläche 1, Horizontalströmungsverteiler 85 und Stopfen 220.

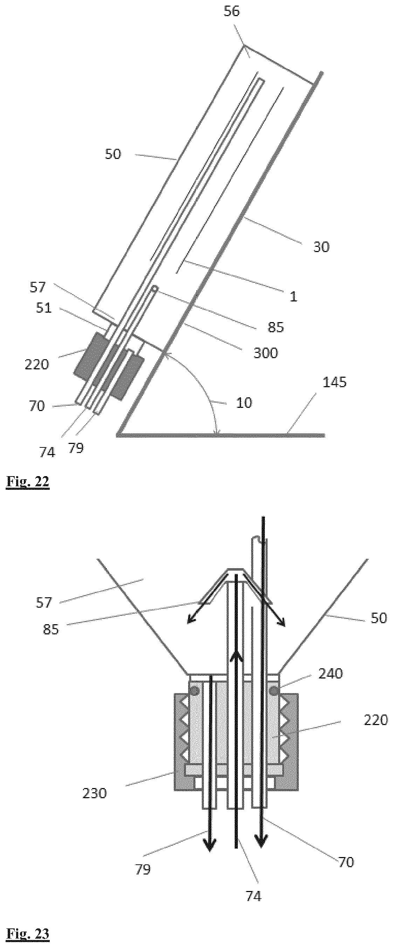

Fig. 22 Seitlicher Schnitt des Flaschenabscheiders nach Fig. 21 auf seinem Gestell.

Fig. 23 Details eines Stopfen 220 mit Überwurf 230 sowie Strömungsverteiler 85 mit nach unten gerichteten Einlassströmungen

Fig 24 Frontansichten eines hängenden Kunststoffbeutels (=BagSettler) als Feststoffabscheiders und seitlicher Schnitt auf Gestell.

Fig. 25 zeigt den Einfluss verschiedener Strömungsverteiler 85 auf die Rückhalteleistung R bei verschiedener effektiver Steiggeschwindigkeit v=q/Aeff.

**Bezugszeichen:**

1 Lamellenpaket / Abscheiderfläche

5 Stegbreite

8 Plattenabstand

10 Winkel

13 Länge

15 Breite

18 Höhe

30 Stützplatte

50 Kunststoffbeutel oder Kunststoffflasche

51 Hals

52 Überschuss / Falte

55 Schweißnaht

56 Erntestromsammelbereich

57 Feststoffsammelbereich

58 Winkel

59 Winkel

60 Befestigungsband

70 Erntestrom (Harvest)

74 Bioreaktorgemisch / Feed

79 Rückführung

80 Durchführung

81 Strömungsinverter

84 Durchführung

85 Strömungsverteiler insb. Horizontalverteiler oder Strömungsverteiler mit nach unten gerichteten Einlassströmungen

86 Einlassströmung
88 zentrale Absaugung
89 Durchführung
90 Anschlussplatte
100 Gehäuse
110 Deckel
112 Verlängerung
115 Befestigungselement
130 Rahmen
140 Gestell
142 Vorsprung
145 Gestellfuß
148 Auflage
200 Vibrator
210 Montageplatte
220 Deckel oder Stopfen
230 Überwurfmutter
240 O-Ring

Profile eines Lamellenpakets

311 Lamellenpaket
320 Rechteckprofil
321 Lamellenpaket
330 Rundprofil
331 Lamellenpaket
340 Rundprofil
341 Lamellenpaket
350 6-Eck-Profil
351 Lamellenpaket

500 Theoritische maximale Abscheiderfläche
501 Abscheiderbereich
510 Einlassfläche

600 Kühlvorrichtung
610 Bioreaktor
620 Abscheidevorrichtung
630, 631 Pumpen
640 Abscheider = Abscheidebeutel + Kühlvorrichtung ggf. im Gestell oder Behälter integriert.
650 Kulturmedium

Im Folgenden werden Untersuchungen der Anwendbarkeit der erfindungsgemäßen Vorrichtungen beschrieben ohne sich hierauf zu beschränken.

**Partikelsystem**

Zur Simulation von Zellen wird das Partikelsystem Polyacrylnitril X-Polymerisat "PAN-X" verwendet. Das in Wasser unlösliche Polymer findet hauptsächlich in der Bekleidungsindustrie zur Herstellung von Fasern Verwendung. Nachfolgend ein Auszug aus dem Produktdatenblatt des Herstellers Firma Dralon GmbH, Dormagen.

| Name | PAN-X |
|---|---|
| Chemische Formel | $[C_3H_3N]_n$ |
| Darreichungsform | Pulver |
| Partikelform | sphärisch |

(fortgesetzt)

| Name | PAN-X |
|---|---|
| Farbe | weiß |
| Dichte | 1,18 g/m$^3$ |
| Korngrößenverteilung | 97 Vol% $\leq$ 50$\mu$m |
| Löslichkeit in Wasser | unlöslich |
| Zündtemperatur | 485 °C |

Die Partikelgrößenverteilung zeigt den häufigsten Partikeldurchmesser zwischen 15 und 30 $\mu$m gemessen mit dem Laserbeugungsmessgerät Mastersizer der Firma Malvern, was etwa eukaryotischen Zellen (CHO, BHK) entspricht.

## Abscheidesysteme

### Schrägkanalabscheider

Untersucht wurden zu Vergleichszwecken ein großer und ein kleiner Plattenabscheider aus Edelstahl nach WO03/020919 mit theoretischen Abscheideflächen von $A_{th}$ = 1,42 m$^2$ bzw. A$_{th}$ = 0.027 m$^2$ als Modell zum erfindungsgemäßen Abscheider nach Fig. 2. Der große Abscheider besitzt 20 Platten, die in einem Abscheidervolumen von 17,4 L untergebracht sind. Der kleine Lamellenabscheider besteht aus 4 Platten in einem Abscheidervolumen von 0,3 L.

### Würfelabscheider

Zwei Würfel wurden als hydrodynamisches Modell mit den Kantenlängen D = 200 mm und D = 400 mm aus Plexiglasplatten hergestellt. Die obere Ecke des Würfels besaß eine Öffnung für die Durchführung von Schlauchleitungen. Es wurde ein Einbau zur Strömungsverteilung (auch (Strömungs)Verteiler 85 genannt) der Partikelsuspension in Form eines an einem Schlauch befestigten T-Stücks oder Y-Stücks (jeweils zwei Einlaufe) bis in die Mitte (h=50 % HK) des Kunststoffmodells eingeführt. Die Breite der Verteiler c wurde variiert. Durch einen weiteren bis in die untere Konusspitze (Feststoffsammelbereich 57 auch Sammler) reichenden Schlauch, wurde das Sediment strömungsinvertierend vertikal nach oben abgezogen. Durch eine weitere Durchführung wurde einen Kunststoffrohr zum Sammeln der Klarphase = Durchführung 80befestigt, so dass die Klarphase (=Erntestrom 70) von der Oberfläche strömungsinvertierend nach unten gerichtet (U-Rohr) abgezogen wurde. Die allmählichen Querschnitterweiterungen zur unteren und oberen Abscheiderspitze erlauben eine gute Strömungsvergleichmäßigung und fungieren dadurch bereits als Strömungssammler (Feststoffsammelbereich 57 unten, Erntestromsammelbereich 56 oben),die auch ohne strömungsinvertierende Einbauten ausreichend gut funktionieren sollten.

### Tetraeder-Abscheider

Der Tetraeder wurde als Plexiglasmodell aus gleichseitigen Dreiecken mit einer Kantenlänge D = 400 mm hergestellt. Gegenüber der Ecke, die als konischen Feststoffsammelbereich 57 ausgewählt wurde, war der Feststoffabscheider nach oben geöffnet Durch die Öffnungen konnten verschiedene Durchführungen für Einleitung und Abzug von Suspension (=Feed 74), Sedimentes und Klarlaufes (=Erntestrom 70) eingebaut werden. An die Durchführungen, in der Regel Schläuche wurden Verteiler und Kunststoffrohr zum Sammeln der Klarphase (= Durchführung 80)analog zum Würfel ausgeführt und positioniert.

Der Tetraeder unterscheidet sich in seiner Strömungs-charakteristik gegenüber dem Würfel in erster Linie darin, dass eine strömungsbegünstigte Verjüngung lediglich nach unten zum Rücklaufsammler vorhanden ist und die Klarphase am Punkt der größten Oberfläche gesammelt wird. Zum gleichmäßigen Abzug wird beim Tetraeder daher ein in dieser maximalen Abscheidefläche platzierter Strömungsverteiler 85 verwendet

Die Abscheidefläche $A_{th}$ betrug bei der untersuchten Einheit 0,069 m$^2$ bei 7,6 L Volumen.

### Vertikalabscheider mit konischen Zulauf -Typ Dortmundbrunnen

Dieser Vertikalabscheider ist häufig in der Abwassertechnik eingesetzt. Der untersuchte Abscheider bestand aus einem zylindrischen Mantel mit einem Querschnitt von 145 mm, der im unteren Bereich einen Sammeltrichter bildet, und im oberen Bereich einen mittig platzierten konischen Zulauf mit einem Querschnitt von 51 mm aufweist. Beide Elemente wurden aus Glas hergestellt. Die ungeklärte Flüssigkeit wurde über den konischen Zulauf von oben in den zylindrischen Bereich (vertikalen Abscheidebereich) eingeleitet und stieg in den Abscheidebereich auf, während die Schwebstoffe in den Sammeltrichter sedimentierten. Am oberen Ende des Abscheidebereichs wurde die Klarphase an vier Punkten gesammelt. Der untersuchte Vertikalabscheider hatte ein Volumen von 1,7 L einer Abscheiderfläche $A_{th}$ von 0,014 m$^2$.

## Methoden

### Analysemethode

Die Probe wird mittels einer Saugnutsche abfiltriert (Porengröße <2$\mu$m), das Filterpapier bei 140 °C getrocknet und gewogen. Hierfür wurde eine Trocknungswaage (Sartorius MA45) verwendet.

### Versuchsdurchführung

PAN-X 3 g/L wurde im Vorlagebehälter bereitgestellt und in den jeweiligen Abscheider mittels Schlauchpumpen (Watson-Marlow Du323) eingeführt. Über die Pumprate der Schlauchpumpen q wird die gewünschte Steiggeschwindigkeit v (v=q/Ath, wobei der q der Erntestrom ist, mit dem der Abscheider bei gegebener Perfusionsrate und Bioreaktorvolumen V belastet wird) eingestellt.

Die Partikelsuspension wird zunächst im Kreis gepumpt. Nach einer Wartezeit von zwei hydrodynamischen Verweilzeiten zur Einstellung stationärer Bedingungen wurde die Probenahme aus dem Erntestrom gestartet.

Das Probevolumen richtete sich nach der Partikelmasse auf dem Filter. Diese sollte zur Messfehlerbegrenzung ca. 100 mg $\pm$ 25 mg betragen. Daraus resultier Probevolumina von 40 bis 800 mL für die Bestimmung der Partikelkonzentration, die per Dreifachbestimmung gemessen wurden.

### Ergebnisse

**Tabelle 1**

| Abscheider | Geometrie | | | R= 1 - $c_H$/c | w = q/Ath | w_eff = w / $\eta$Do |
|---|---|---|---|---|---|---|
| | | | | 1 | m/h | m/h |
| Tetraeder | A | m$^2$ | 0.0692 | 0.91 | 0.03 | 0.0294 |
| | d | mm | 400 | 0.82 | 0.05 | 0.0588 |
| | VS | L | 6.42 | 0.74 | 0.10 | 0.1176 |
| | $\eta$Do | - | 0.8 | 0.51 | 0.20 | 0.2353 |
| Würfel 1 | A | m$^2$ | 0.052 | 0.96 | 0.03 | 0.0208 |
| | d | mm | 200 | 0.92 | 0.05 | 0.0417 |
| | VS | L | 8.00 | 0.83 | 0.10 | 0.0833 |
| | $\eta$Do | - | 1.2 | 0.63 | 0.20 | 0.1667 |
| Würfel 2 | A | m$^2$ | 0.208 | 0.95 | 0.03 | 0.0208 |
| | d | mm | 400 | 0.91 | 0.05 | 0.0417 |
| | VS | L | 64 | 0.81 | 0.10 | 0.0833 |
| | $\eta$Do | - | 1.2 | 0.61 | 0.20 | 0.1667 |
| PLA 1 | A | m$^2$ | 0.0274 | 0.95 | 0.03 | 0.0227 |
| | Z | 1 | 4 | 0.90 | 0.05 | 0.0455 |
| | VS | L | 0.375 | 0.76 | 0.10 | 0.0909 |
| | $\eta$Do | - | 1.1 | 0.61 | 0.20 | 0.1818 |
| PLA 2 | A | m$^2$ | 1.42 | 0.96 | 0.03 | 0.0227 |
| | Z | - | 21 | 0.92 | 0.05 | 0.0455 |
| | VS | L | 18 | 0.79 | 0.10 | 0.0909 |
| | $\eta$Do | - | 1.1 | 0.59 | 0.20 | 0.1818 |

(fortgesetzt)

| Abscheider | Geometrie | | | R= 1 - $c_H$/c | w = q/Ath | w_eff = w / $\eta$Do |
|---|---|---|---|---|---|---|
| | | | | 1 | m/h | m/h |
| Dortmundbrunnen | A | m$^2$ | 0.0142 | 0.93 | 0.03 | 0.9286 |
| | d | mm | 145 | 0.87 | 0.05 | 0.8704 |
| | VS | L | 1.7 | 0.75 | 0.10 | 0.7506 |
| | $\eta$Do | - | 1 | 0.56 | 0.20 | 0.5617 |

Der Vergleich der Abscheidesysteme zeigt das erwartete Sinken des Rückhaltgrades R mit steigendem Medien-bzw. Erntestrom q bzw. der effektiven Steiggeschwindigkeit v=q/$A_{eff}$ (Fig. 19).

Die effektive Steiggeschwindigkeit ergibt sich durch Einführung des Wirkungsgrades $\eta$1, der die unterschiedliche Rückhalteleistung der verwendeten Maximalfläche der Abscheider gegenüber dem Dortmundbrunnen ausweist. Fig 19 zeigt, dass die Abscheider nach Hinzunahme dieses Wirkungsgrades durch einen gemeinsame. Ausgleichgrade beschreibbar sind.

Die Leistungsfähigkeit der Abscheider, ist in Fig 20 gegenübergestellt. Diese Darstellung zeigt, wie viel Abscheidervolumen notwendig ist, um die effektive Abscheidefläche unterzubringen. Kleine Abscheidervolumina sind bei Zellkulturen wünschenswert, um Aufenthaltszeiten außerhalb des versorgten Fermentationsraumes zu minimieren. Sehr gut schneiden bei dieser Gegenüberstellung die Schrägkanalabscheider ab, die unabhängig vom Maßstab mit sehr hohen Abscheideflächen pro Abscheidervolumen größer 50 l/m betreibbar sind. Dieses Beispiel macht die hervorragende Skalierbarkeit dieser Abscheidersysteme deutlich. Im Gegensatz dazu wird bei den Vertikalabscheidern erheblich mehr Volumen benötigt, um in diesen die horizontale Abscheidefläche zu entfalten. Außerdem sinkt die Effizienz der Unterbringung bei der Maßstabsvergrößerung mit V = A$^{3/2}$ ab. Überraschenderweise ist die Effizienz der Einwegmodelle, der Würfel und des Tetraeders, dem Standardsystem des Dortmundbrunnens deutlich überlegen, so dass diese sehr einfachen und preiswerten Systeme in erheblich größeren Bioreaktoren (bis zu ca. 6- fach) als der Dortmundbrunnen eingesetzt werden können.

Eine weitere Anpassung (Geometrie und / oder Position) der Zulaufverteiler und Erntestromsammler könnten zu einer Optimierung der Rückhaltegrads R führen.

**Die Arbeiten, die zu dieser Anmeldung geführt haben, wurden gemäß der Finanzhilfevereinbarung "Bio.NRW: MoBiDik - Modulare Bioproduktion - Disposable und Kontinuierlich" (Förderkennzeichen w1004ht022a) im Rahmen des Europäischen Fonds für regionale Entwicklung (EFRE) gefördert.**

**Patentansprüche**

1. Feststoffabscheider zur Rückhaltung und Rückführung von Feststoffen aus einem Reaktorgemisch, umfassend einen durchströmten sterilisierbaren Kunststoffbeutel oder Kunststofflasche (50) und innerhalb des Kunststoffbeutels oder Kunststofflasche (50):

   - im oberen Bereich ein oder mehrere Durchführungen/Einbauten (80, 81) zum Abziehen eines von den Feststoffen getrennten Erntestroms (70) aus einem Erntestromsammelbereich (56),
   - im oberen Segment eines mittleren Bereiches einen Abscheidebereich (1, 501) mit einer Abscheidefläche, die während des Betriebs mit einen Winkel (10) von 0° bis 80° zur Horizontalen geneigt ist,
   - im unteren Segment des mittleren Bereiches eine oder mehrere Durchführungen oder Einbauten (84, 85) zur Strömungsverteilung des Reaktorgemisches (74),
   - im unteren Bereich einen unten verjüngten Feststoffsammelbereich (57) zum Sammeln der Feststoffe mit Hilfe der Schwerkraft.

2. Feststoffabscheider nach Anspruch 1, wobei Feststoffsammelbereich (57) eine oder mehrere Durchführungen (89) oder Einbauten (88) zum Abziehen der Feststoffe aufweist.

3. Feststoffabscheider nach einem der Ansprüche 1 oder 2, wobei der Feststoffsammelbereich (57) konisch oder pyramidal nach unten verjüngt ist.

4. Feststoffabscheider nach einem der Ansprüche 1 bis 3, der mindestens einen Einwegsensor im Innenraum umfasst.

5. Feststoffabscheider nach einem der Ansprüche 1 bis 4, wobei der Abscheidebereich aus einer Vielzahl von nebeneinander angeordneten Kanälen in einem Lamellenpaket (1) besteht und die Abscheidefläche während des Betriebs mit einem Winkel (10) von 30° bis 80° zur Horizontalen geneigt ist.

6. Feststoffabscheider nach Anspruch 5, wobei das Lamellenpaket (1) aus mehreren aufeinander gestapelten Stegplatten besteht, die die Kanäle des Lamellenpakets (1) bilden.

7. Feststoffabscheider nach einem der Ansprüche 5 oder 6, wobei die Kanäle eine Kanallänge L von 30 % bis 95% einer Länge LK des Kunststoffbeutels oder Kunststofflasche aufweisen.

8. Feststoffabscheider nach einem der Ansprüche 5 bis 7, wobei das Verhältnis von Steghöhe zur Kanalbreite hs/$d$ $0,01 \leq hs/d \leq 5$ beträgt, mit der Einschränkung.

9. Feststoffabscheider nach einem der Ansprüche 1 bis 4 , wobei der Kunststoffbeutel (50) polyeder- oder kegelförmig ist und wobei der Kunststoffbeutel (50) während des Betriebs so gestellt ist, dass der unten verjüngte Feststoffsammelbereich 57 durch die Wände des Kunststoffbeutels und die Spitze bzw. Ecke des Polyeders oder des Kegels gebildet ist.

10. Feststoffabscheider nach Anspruch 9, wobei der Kunststoffbeutel (50) ein Disphenoid, eine gerade Pyramide, ein Oktaeder oder ein Würfel ist.

11. Feststoffabscheider nach einem der Ansprüche 1 bis 4, wobei der Kunststoffbeutel oder Kunststoffflasche (50) einen viereckigen Querschnitt aufweist, wobei der nach unten verjüngte Feststoffsammelbereich 57 in einem Hals endet, der mit einem Deckel oder Stopfen verschlossen ist, wobei der Deckel oder Stopfen alle Durchführungen 80, 84, 89 aufweist.

12. Feststoffabscheider nach einem der Ansprüche 1 bis 11 umfassend einen Behälter zur Aufnahme des Kunststoffbeutels 50, wobei der Behälter mindestens:

   - einen Innenraum zur Aufnahme des Kunststoffbeutels 50, wobei der Innenraum an die Form des Kunststoffbeutels 50 mittels an die Form des Kunststoffbeutels 50 angepasste Wände, die den Innenraum einschließen und von der Außenwelt abgrenzen, angepasst ist, eine Öffnung zur Einführung des Kunststoffbeutels 50 von oben in den Behälter,

   umfasst.

13. Bioreaktoranlage umfassend einen Bioreaktor verbunden mit einem Feststoffabscheider nach einem der Ansprüche 1 bis 12.

14. Verfahren zur Rückhaltung und Rückführung von Feststoffen, insbesondere Zellen, in einem Feststoffabscheider nach einem der Ansprüche 1 bis 12 umfassend ein durchströmtes Gefäß, wobei dem Gefäß kontinuierlich oder absatzweise feststoffenthaltendes Medium zugeführt und feststofffreies Medium abgeführt wird, **dadurch gekennzeichnet, dass** das Gefäß ein durchströmter sterilisierbarer Kunststoffbeutel oder - flasche ist, der im unteren Bereich schräg angestellte Flächen, günstiger Weise einen unten konisch verjüngten Feststoffsammelbereich (57) zum Sammeln der Feststoffe mit Hilfe der Schwerkraft aufweist.

**Claims**

1. Solids separator for retaining and recirculating solids from a reactor mixture, comprising a flow-bearing sterilizable plastic bag or plastic bottle (50) and, within the plastic bag or plastic bottle (50):

   - in the upper region, one or more passages/internals (80, 81) for withdrawing from a harvest stream collection region (56) a harvest stream (70) that is separated from the solids,
   - in the upper segment of a central region, a separation region (1, 501) having a separation area which during operation is inclined at an angle (10) of 0° to 80° to the horizontal,
   - in the lower segment of the central region, one or more passages or internals (84, 85) for flow distribution of the reactor mixture (74),

- in the lower region, a solids collection region (57) that is tapered at the bottom for collecting the solids using gravity.

2. Solids separator according to Claim 1, wherein solids collection region (57) has one or more passages (89) or internals (88) for withdrawing the solids.

3. Solids separator according to either of Claims 1 and 2, wherein the solids collection region (57) is tapered downwards conically or pyramidally.

4. Solids separator according to any one of Claims 1 to 3, which comprises at least one single-use sensor in the interior.

5. Solids separator according to any one of Claims 1 to 4, wherein the separation region consists of a multiplicity of adjacently arranged channels in a lamellae pack (1) and the separation area during operation is inclined at an angle (10) of 30° to 80° to the horizontal.

6. Solids separator according to Claim 5, wherein the lamellae pack (1) consists of a plurality of ridgeplates stacked one above the other which form the channels of the lamellae pack (1).

7. Solids separator according to either of Claims 5 and 6, wherein the channels have a channel length L of 30% to 95% of a length LK of the plastic bag or plastic bottle.

8. Solids separator according to any one of Claims 5 to 7, wherein the ratio of ridge height to the channel width hs/d is $0.01 \leq hs/d \leq 5$ with the restriction.

9. Solids separator according to any one of Claims 1 to 4, wherein the plastic bag (50) is polyhedral or conical and wherein the plastic bag (50), during operation, is placed such that the solids collection region (57) that is tapered at the bottom is formed by the walls of the plastic bag and the apex or corner of the polyhedron or of the cone.

10. Solids separator according to Claim 9, wherein the plastic bag (50) is a disphenoid, a regular pyramid, an octahedron or a cube.

11. Solids separator according to any one of Claims 1 to 4, wherein the plastic bag or plastic bottle (50) has a rectangular cross section, wherein the downwardly tapered solids collection region (57) ends in a neck which is closed with a cover or stopper, wherein the cover or stopper has all passages (80, 84, 89).

12. Solids separator according to any one of Claims 1 to 11, comprising a container for receiving the plastic bag (50), wherein the container comprises at least:

- an interior for receiving the plastic bag (50), wherein the interior is adapted to the shape of the plastic bag (50) by means of walls which are adapted to the shape of the plastic bag (50) and enclose the interior and delimit it from the exterior, an opening for introducing the plastic bag (50) from the top into the container.

13. Bioreactor system comprising a bioreactor connected to a solids separator according to any one of Claims 1 to 12.

14. Method for retaining and recirculating solids, in particular cells, in a solids separator according to any one of Claims 1 to 12 comprising a flow-bearing vessel, wherein solids-containing medium is supplied continuously or batchwise to the vessel, and solids-free medium is removed from the vessel, **characterized in that** the vessel is a flow-bearing sterilizable plastic bag or plastic bottle which, in the lower region, has faces set at an incline, favorably a solids collection region (57) that is tapered conically at the bottom for collecting the solids using gravity.

**Revendications**

1. Séparateur de solides pour la rétention et le recyclage de solides à partir d'un mélange réactionnel, comprenant un sac en matière plastique ou une bouteille en matière plastique (50) stérilisable traversé(e) et, à l'intérieur du sac en matière plastique ou de la bouteille en matière plastique (50) :

- dans la zone supérieure, un ou plusieurs passages/composants (80, 81) pour le soutirage d'un courant de récolte (70) séparé des solides à partir d'une zone de collecte du courant de récolte (56),

- dans le segment supérieur d'une zone centrale, une zone de séparation (1, 501) comprenant une surface de séparation, qui est inclinée d'un angle (10) de 0° à 80° par rapport à l'horizontale pendant le fonctionnement,
- dans le segment inférieur de la zone centrale, un ou plusieurs passages ou composants (84, 85) pour la répartition de l'écoulement du mélange réactionnel (74),
- dans la zone inférieure, une zone de collecte de solides effilée (57) en bas pour la collecte des solides à l'aide de la force de pesanteur.

**2.** Séparateur de solides selon la revendication 1, dans lequel la zone de collecte de solides (57) comprend un ou plusieurs passages (89) ou un ou plusieurs composants (88) pour le soutirage des solides.

**3.** Séparateur de solides selon l'une quelconque des revendications 1 ou 2, dans lequel la zone de collecte de solides (57) est effilée vers le bas sous forme conique ou pyramidale.

**4.** Séparateur de solides selon l'une quelconque des revendications 1 à 3, qui comprend au moins un capteur à usage unique dans l'espace intérieur.

**5.** Séparateur de solides selon l'une quelconque des revendications 1 à 4, dans lequel la zone de séparation est constituée par une pluralité de canaux agencés les uns à côté des autres dans un paquet de lamelles (1), et la surface de séparation est inclinée d'un angle (10) de 30° à 80° par rapport à l'horizontale pendant le fonctionnement.

**6.** Séparateur de solides selon la revendication 5, dans lequel le paquet de lamelles (1) est constitué par plusieurs plaques alvéolaires empilées les unes au-dessus des autres, qui forment les canaux du paquet de lamelles (1).

**7.** Séparateur de solides selon l'une quelconque des revendications 5 ou 6, dans lequel les canaux présentent une longueur de canal L de 30 % à 95 % d'une longueur LK du sac en matière plastique ou de la bouteille en matière plastique.

**8.** Séparateur de solides selon l'une quelconque des revendications 5 à 7, dans lequel le rapport entre la hauteur des alvéoles et la largeur du canal hs/d est selon $0,01 \leq hs/d \leq 5$, avec la restriction.

**9.** Séparateur de solides selon l'une quelconque des revendications 1 à 4, dans lequel le sac en matière plastique (50) est de forme polyédrique ou conique, et dans lequel le sac en matière plastique (50) est positionné pendant le fonctionnement de telle sorte que la zone de collecte de solides effilée en bas (57) soit formée par les parois du sac en matière plastique et la pointe ou le coin du polyèdre ou du cône.

**10.** Séparateur de solides selon la revendication 9, dans lequel le sac en matière plastique (50) est un disphénoïde, une pyramide droite, un octaèdre ou un cube.

**11.** Séparateur de solides selon l'une quelconque des revendications 1 à 4, dans lequel le sac en matière plastique ou la bouteille en matière plastique (50) présente une section quadrilatérale, la zone de collecte de solides effilée vers le bas (57) finissant dans un col qui est fermé avec un couvercle ou un bouchon, le couvercle ou le bouchon comprenant tous les passages 80, 84, 89.

**12.** Séparateur de solides selon l'une quelconque des revendications 1 à 11, comprenant un contenant pour la réception du sac en matière plastique 50, le contenant comprenant au moins :

- un espace intérieur pour la réception du sac en matière plastique (50), l'espace intérieur étant adapté à la forme du sac en matière plastique (50) au moyen de parois adaptées à la forme du sac en matière plastique (50), qui entourent l'espace intérieur et le délimitent de l'extérieur, une ouverture pour l'insertion du sac en matière plastique (50) depuis le haut dans le contenant.

**13.** Unité de bioréacteur comprenant un bioréacteur raccordé avec un séparateur de solides selon l'une quelconque des revendications 1 à 12.

**14.** Procédé de rétention et de recyclage de solides, notamment de cellules, dans un séparateur de solides selon l'une quelconque des revendications 1 à 12, comprenant un récipient traversé, un milieu contenant des solides étant introduit et un milieu exempt de solides étant déchargé de manière continue ou discontinue du récipient, **caractérisé**

**en ce que** le récipient est un sac ou une bouteille en matière plastique stérilisable traversé(e), qui comprend dans la zone inférieure des surfaces disposées en biais, avantageusement une zone de collecte de solides effilée coniquement en bas (57) pour la collecte des solides à l'aide de la force de pesanteur.

**Fig. 1**

**Fig. 2**

**Fig. 3**

Fig. 4

**Fig. 5**

**Fig. 6**

**Fig. 7**

**Fig. 8**

**Fig. 9**

**Fig. 10**

Fig. 11

Fig. 12

**Fig. 13**

**Fig. 14**

70    74    79

56

500

80

89

81    84

86

50    85

57

88

**Fig. 15**

74    70

79

80

90

56

510

510    81

57

88

**Fig. 16**

**Fig. 17**

**Fig. 18**

$$R = 1 - c_H/c$$

| | Abscheider | $\eta 1$ |
|---|---|---|
| △ | Tetraeder | 0.8 |
| ◇ | Würfel 1 | 1.2 |
| ◈ | Würfel 2 | 1.2 |
| □ | PLA 1 | 1.1 |
| ▣ | PLA 2 | 1.1 |
| ○ | Do-Brunn | 1.0 |

$$w_{eff} = q / A_{eff} = \eta 1 \quad q / A_{th}$$

**Fig. 19**

Schrägkanalabscheiderr

Würfel, Tetraeder

Dortmundbrunnen

| | Abscheider | $\eta 1$ |
|---|---|---|
| △ | Tetraeder | 0.8 |
| ◇ | Würfel 1 | 1.2 |
| ◈ | Würfel 2 | 1.2 |
| □ | PLA 1 | 1.1 |
| ▣ | PLA 2 | 1.1 |
| ○ | Do-Brunn | 1.0 |

$$A_{eff} = \eta 1 \quad A_{th}$$

**Fig. 20**

32

**Fig. 21**

56

50

57
51

220

70

74  79

85
300

30

1

145
10

**Fig. 22**

57
85

50

240

220

230

79

74

70

**Fig. 23**

**Fig. 24**

**Fig. 25**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5817505 A
- EP 0699101 B1
- WO 2003020919 A2
- DE 1022353
- WO 2009152990 A2
- US 6186932 B1
- US 20090180933 A
- WO 03020919 A

**In der Beschreibung aufgeführte Nicht-Patentliteratur**

- **H.-J. BINDER.** Sedimentation aus Ein- und Mehrkornsuspensionen in schräg stehenden, laminar durchströmten Kreis- und Rechteckrohren. *Dissertation Berlin,* 1980